# EUROPÄISCHE PATENTANMELDUNG

(11) **EP 4 080 192 A1**
(43) Veröffentlichungstag der Anmeldung: **26.10.2022**
(21) Anmeldenummer: 22167777.6
(22) Anmeldetag: 12.04.2022
(51) Int. Cl.: G01N 21/05, A61B 5/08, G01N 21/3504, G01N 33/497, A61B 5/097, G01N 21/85

(54) **ADAPTER**

(30) Priorität: 20.04.2021 DE 102021002052
(71) Anmelder: Löwenstein Medical Technology S.A., 2557 Luxembourg (LU)
(72) Erfinder: Kremeier, Peter, 76149 Karlsruhe (DE)
(74) Vertreter: Marx, Thomas

(57) **Zusammenfassung**

Die Erfindung betrifft einen Adapter zur Verwendung mit Spektrometern für die Analyse von Gasen mit einem Gehäuse, das einen Grundkörper, eine Aufnahmeeinrichtung zur Aufnahme eines Spektrometers, einen Gehäuseeingang und einen Gehäuseausgang umfasst, wobei der Gehäuseeingang eine Eintrittsöffnung und der Gehäuseausgang eine Austrittsöffnung umfasst, und mit einem Durchgangskanal, der zwischen der Eintrittsöffnung und der Austrittsöffnung angeordnet ist und wobei der Grundkörper zwischen dem Gehäuseeingang und dem Gehäuseausgang angeordnet ist dadurch gekennzeichnet, dass der Grundkörper mindestens zwei Öffnungen aufweist, die beidseitig des Durchgangskanals an sich gegenüberliegen Seiten angeordnet sind.

## Beschreibung

Die Erfindung betrifft einen Adapter der im Oberbegriff des Anspruchs 1 genannten Art. Insbesondere betrifft die Erfindung einen Adapter zur Verwendung mit Spektrometern, beispielsweise Infrarot-Spektrometern (IR-Spektrometern) für die Analyse von Gasen.

Adapter zur Verwendung mit IR-Spektrometern finden im Bereich der Medizintechnik Verwendung, beispielsweise im Bereich der Beatmung. Der Adapter kann auch als Küvette bezeichnet werden. Die Begriffe Adapter und Küvette werden hierin als Synonyme verwendet.

Die Analyse von Atemgasen kann derart durchgeführt werden, dass die von einem Patienten ausgeatmete Luft durch einen Adapter geleitet wird. Der Adapter ist üblicherweise mit Fenstern für den Durchtritt eines IR- oder Lichtstrahl ausgestattet, die aus licht- und / oder infrarottransparentem Materialien bestehen. In der Medizin werden Einweg- und Mehrwegadapter für Hauptströmungs-Infrarotspektrometer verwendet. Wiederverwendbare Adapter müssen verschiedenen Arten der Sterilisation standhalten und dürfen dabei ihre Eigenschaften nicht verlieren.

Für wiederverwendbare Adapter werden üblicherweise Saphirfenster verwendet, die eine hohe Stabilität aufweisen und für den Infrarotbereich des elektromagnetischen Spektrums transparent sind. Kunststofffenster sind in der Regel weniger stabil und verändern ihre Eigenschaften während der Sterilisation. Daher werden Kunststofffenster nur in Einwegadaptern verwendet.

Verschiedene Arten der Sterilisation können verwendet werden, um wiederverwendbare Adapter zu sterilisieren, insbesondere thermische (Dampf oder trockene Hitze) oder chemische. Dies sind die häufigsten Arten der Sterilisation und haben die schädlichsten Auswirkungen auf das Material des Geräts.

Die Einwirkung von Wasserdampf und hoher Temperatur führt zur Schwächung und Zerstörung von Klebstoff und anderen Arten von Verbindungen verschiedener Materialien. Der Effekt der Wärmeausdehnung ist stärker, wenn die Verbindung mehrere Arten von Materialien enthält. In Kombination mit hoher Temperatur und hohem Druck dringt Wasserdampf in die Klebstoffschicht ein und verändert deren Eigenschaften, was zu Verletzungen der Integrität des Adapters und der Undurchlässigkeit führt.

Die chemische Sterilisation erfolgt mit aggressiven Lösungen, die auch Klebemassen schädigen. In dieser Hinsicht ist die Befestigung des Fensters an dem Adaptergehäuse ein kritischer Schritt im Herstellungsprozess, insbesondere in Fällen, in denen der Adapter aus einem anderen Material als das Fenster hergestellt ist.

In JP 3677672 B2 wird eine Adapterkonstruktion zur Messung der Konzentration von Gaskomponenten nach dem spektrometrischen Verfahren vorgeschlagen, die darin besteht, zusammen mit einem transparenten Fensterrahmen die Bildung von Tropfen kondensierter Flüssigkeit aus dem Luftstrom an der inneren Fensterfläche zu verhindern. Der Nachteil dieser technischen Lösung ist die Änderung der Querschnittsfläche des Luftweges der Küvette, die in der Folge zu einer Erhöhung der Konzentrationsanstiegszeit führt.

US 2016/0184545 A1 offenbart ein Adaptergussverfahren, dass es ermöglicht, einen Adapter zu erhalten, der einen Weg für einen Luftstrom und integrierte Fenster enthält, die Messungen des Luftstroms durch den Adapter ermöglichen. Das Gießen erfolgt einstufig, wobei zur Erzielung der erforderlichen Fensterdicke, die geringer ist als die normale Dicke der Adapterwände, Stifte verwendet werden, mit denen die zuvor geformte Fensterfläche auf die erforderliche Dicke gedrückt wird. Die Fenster befinden sich auf gegenüberliegenden Seiten des Luftwegs des Adapters.

Adapter und Fenster bilden eine einteilige Konstruktion und die Fenster bestehen aus demselben Material wie das Gehäuse des Adapters. Insofern entsprechen die optischen Eigenschaften des Fensters den optischen Eigenschaften des Gehäusematerials. In den meisten Fällen ist es nicht angebracht, das Gerät aus demselben Material herzustellen, da dies zu zusätzlichen Kosten führt. Produktionstechnisch wird davon ausgegangen, dass das Gerätegehäuse aus polymeren Kunststoffen besteht. Die optischen Eigenschaften solcher Fenster sind jedoch schlechter als die optischen Eigenschaften von beispielsweise Saphirfenstern.

In US 7,629,039 B2 wird eine Fensterkonstruktion zur Verwendung in einem Adapter für einen Infrarot-Gasanalysator zur Analyse der ausgeatmeten Luft vorgeschlagen, wobei das Gas durch den Durchgang im Adapter mit einem Fenster strömt, das sich auf einander gegenüberliegenden Seiten des Kanals befindet, so dass der ein Infrarotstrahl durch das Fenster und den Kanal gerichtet werden kann, der das spezifizierte ausgeatmete Gas enthält. Das Fenster ist in Form einer festen Kunststoffstruktur hergestellt und hat eine im Wesentlichen kreisförmige Form und umfasst eine umgebende Kante und einen zentralen Teil. In Bezug auf die spezifizierte Kante vertieft und ein Fenster bildend, durch das der Durchgang von Infrarotstrahlen sichergestellt ist.

Der Nachteil dieser technischen Lösung ist das Vorhandensein eines Hohlraums zwischen dem vertieften Fenster des Adapters und dem Gasanalysator, der beim Einbau des Adapters in den Gasanalysator durch versehentliches Ausatmen von medizinischem Personal Kohlendioxid erhalten kann. Dieses Kohlendioxid erhöht einen Fehler im Messergebnis.

Die Aufgabe der Erfindung besteht darin, einen Adapter bereitzustellen, der einfach und kostengünstig in der Herstellung ist und dennoch zuverlässige Messergebisse liefert.

Diese Aufgabe wird erfindungsgemäß gelöst durch einen Adapter, der die kennzeichnenden Merkmale des Anspruchs 1 aufweist. Die Unteransprüche betreffen vorteilhafte Ausgestaltungen der Erfindung.

Die Erfindung betrifft einen Adapter zur Verwendung mit Spektrometern für die Analyse von Gasen mit einem Gehäuse, das einen Grundkörper, eine Aufnahmeeinrichtung zur Aufnahme eines Spektrometers, einen Gehäuseeingang und einen Gehäuseausgang umfasst, wobei der Gehäuseeingang eine Eintrittsöffnung und der Gehäuseausgang eine Austrittsöffnung umfasst, und mit einem Durchgangskanal, der zwischen der Eintrittsöffnung und der Austrittsöffnung angeordnet ist und wobei der Grundkörper zwischen dem Gehäuseeingang und dem Gehäuseausgang angeordnet ist. Erfindungsgemäß weist der Grundkörper mindestens zwei Öffnungen auf, die beidseitig des Durchgangskanals an sich gegenüberliegen Seiten angeordnet sind.

In manchen Ausführungsformen ist der Adapter dadurch gekennzeichnet, dass der Grundkörper zwei Seitenwände mit jeweils einer Öffnung aufweist, durch die eine Strahlung derart gerichtet werden kann, dass die Strahlung den Durchgangskanal schneidet.

In manchen Ausführungsformen ist der Adapter dadurch gekennzeichnet, dass die Strahlung eine Infrarot-Strahlung ist.

In manchen Ausführungsformen ist der Adapter dadurch gekennzeichnet, dass der Durchgangskanal ausgebildet und eingerichtet ist, Atemgase zu führen.

In manchen Ausführungsformen ist der Adapter dadurch gekennzeichnet, dass der Adapter eine maximale Länge aufweist, wobei sich der Durchgangskanal von der Eintrittsöffnung zu der Austrittsöffnung über die maximale Länge des Adapters erstreckt.

In manchen Ausführungsformen ist der Adapter dadurch gekennzeichnet, dass die Öffnungen in ihrer Grundform ein Polygon sind, bevorzugt ein Viereck.

In manchen Ausführungsformen ist der Adapter dadurch gekennzeichnet, dass mindestens eine der mindestens zwei Öffnungen zur Herstellung eines Betriebsmodus mit mindestens einer Abdeckung abgedeckt werden.

In manchen Ausführungsformen ist der Adapter dadurch gekennzeichnet, dass Adapter und Abdeckung einstückig oder zweistückig ausgeführt sind.

In manchen Ausführungsformen ist der Adapter dadurch gekennzeichnet, dass Adapter und Abdeckung aus demselben Material oder aus unterschiedlichen Materialien bestehen.

In manchen Ausführungsformen ist der Adapter dadurch gekennzeichnet, dass Adapter und Abdeckung zweistückig ausgeführt und aus demselben Materialien gefertigt sind.

In manchen Ausführungsformen ist der Adapter dadurch gekennzeichnet, dass der Adapter aus einem Kunststoff gefertigt ist, bevorzugt aus einem thermoplastischen Kunststoff.

In manchen Ausführungsformen ist der Adapter dadurch gekennzeichnet, dass der Adapter aus einem Polycarbonat (PC), einem Polymethylmethacrylat (PMMA), einem Polystyrol (PS) oder einem Cycloolefin-Copolymer (COC, Topas) hergestellt ist.

In manchen Ausführungsformen ist der Adapter dadurch gekennzeichnet, dass der Adapter aus PMMA Plexiglas 7N, Topas 6017-S04, PC Lexan 121 RM1, PC Makrolon 2400, PS Styrolution 124 L, Topas 8007 oder COC 8007 X-04, bevorzugt aus COC oder PMMA hergestellt ist.

In manchen Ausführungsformen ist der Adapter dadurch gekennzeichnet, dass die Abdeckung für Strahlung, insbesondere IR-Strahlung, durchlässig ist. In manchen Ausführungsformen ist der Adapter dadurch gekennzeichnet, dass die Abdeckung eine Durchlässigkeit für Infrarotstrahlung von mindestens 0,5 aufweist.

In manchen Ausführungsformen ist der Adapter dadurch gekennzeichnet, dass die Abdeckung aus einem Kunststoff hergestellt ist.

In manchen Ausführungsformen ist der Adapter dadurch gekennzeichnet, dass die Abdeckung aus einem Polycarbonat (PC), einem Polymethylmethacrylat (PMMA), einem Polystyrol (PS) oder einem Cycloolefin-Copolymer (COC, Topas) hergestellt ist.

In manchen Ausführungsformen ist der Adapter dadurch gekennzeichnet, dass die Abdeckung aus PMMA Plexiglas 7N, Topas 6017-S04, PC Lexan 121 RM1, PC Makrolon 2400, PS Styrolution 124 L, Topas 8007 oder COC 8007 X-04, bevorzugt aus COC 8007 X-04 hergestellt ist.

In manchen Ausführungsformen ist der Adapter dadurch gekennzeichnet, dass die Abdeckung eine Folie ist.

In manchen Ausführungsformen ist der Adapter dadurch gekennzeichnet, dass die Folie eine Dicke zwischen 2000 µm und 50 µm, bevorzugt zwischen 1000 µm und 100 µm, besonders bevorzugt zwischen 600 µm und 100 µm, beispielsweise 140 µm aufweist.

In manchen Ausführungsformen ist der Adapter dadurch gekennzeichnet, dass die Folie größer ist als die Öffnung.

In manchen Ausführungsformen ist der Adapter dadurch gekennzeichnet, dass der Grundkörper zwei Seitenwände umfasst, die jeweils eine Öffnung, einen inneren Rand, einen äußeren Rand und eine Seitenfläche umfassen.

In manchen Ausführungsformen ist der Adapter dadurch gekennzeichnet, dass die Folie mindestens bereichsweise auf die Seitenwände aufgebracht wird und mindestens eine der mindestens zwei Öffnungen zur Herstellung eines Betriebsmodus des Adapters vollständig abdeckt.

In manchen Ausführungsformen ist der Adapter dadurch gekennzeichnet, dass die Folie die Öffnungen derart abdeckt, dass der Durchgangskanal luftdicht abgeschlossen ist und ein Durchgang von Gasen ausschließlich von dem Gehäuseeingang zu dem Gehäuseausgang möglich ist und umgekehrt.

In manchen Ausführungsformen ist der Adapter dadurch gekennzeichnet, dass die Folie verklebt, verschweißt und/oder mechanisch verrastet mit der Seitenwand oder Teilen der Seitenwand verbunden wird.

In manchen Ausführungsformen ist der Adapter dadurch gekennzeichnet, dass 20 % bis 80 % der Folie mit der Seitenwand oder mit Teilen der Seitenwand verbunden werden, bevorzugt 30 % bis 60 %, besonders bevorzugt 40 % bis 50 %.

In manchen Ausführungsformen ist der Adapter dadurch gekennzeichnet, dass die Folie zumindest bereichsweise mit dem inneren Rand und/oder dem äußeren Rand und/oder der Seitenfläche verbunden wird.

In manchen Ausführungsformen ist der Adapter dadurch gekennzeichnet, dass die Folie und der innere Rand und/oder der äußeren Rand und/oder die Seitenfläche über Laserschweißen verbunden werden.

In manchen Ausführungsformen ist der Adapter dadurch gekennzeichnet, dass die Folie eine polygone Form aufweist, bevorzugt eine viereckige Form.

In manchen Ausführungsformen ist der Adapter dadurch gekennzeichnet, dass die Folie Seitenlängen und mindestens eine abgerundete Ecke, bevorzugt zwei abgerundete Ecken umfasst.

In manchen Ausführungsformen ist der Adapter dadurch gekennzeichnet, dass die Seitenlängen der Folie gerade oder gebogen ausgebildet sind, bevorzugt gerade.

In manchen Ausführungsformen ist der Adapter dadurch gekennzeichnet, dass die Seitenlängen der Folie eine Länge von 5 mm bis 60 mm aufweisen, bevorzugt von 10 mm bis 30 mm, besonders bevorzugt von 15 bis 20 mm.

In manchen Ausführungsformen ist der Adapter dadurch gekennzeichnet, dass die Aufnahmevorrichtung zur Aufnahme eines Spektrometers benachbart zu dem Grundkörper liegt.

In manchen Ausführungsformen ist der Adapter dadurch gekennzeichnet, dass die Aufnahmevorrichtung einstückig mit dem Gehäuse ausgebildet ist.

In manchen Ausführungsformen ist der Adapter dadurch gekennzeichnet, dass die Aufnahmevorrichtung einen Aufnahme-Boden und zwei Aufnahme-Seitenwände umfasst, innerhalb derer der Grundkörper angeordnet ist.

In manchen Ausführungsformen ist der Adapter dadurch gekennzeichnet, dass die Aufnahme-Seitenwände und der Aufnahmeboden breiter sind als der Grundkörper tief ist. In manchen Ausführungsformen ist der Adapter dadurch gekennzeichnet, dass die Aufnahme-Seitenwände höher sind als der Grundkörper hoch ist.

In manchen Ausführungsformen ist der Adapter dadurch gekennzeichnet, dass die Aufnahmevorrichtung eingerichtet und ausgebildet ist, ein formkomplementäres IR-Spektrometer derart zwischen die zwei Aufnahme-Seitenwände und den Aufnahme-Boden aufzunehmen, dass zumindest die mindestens zwei Öffnungen des Grundkörpers vom IR-Spektrometer umschlossen sind.

In manchen Ausführungsformen ist der Adapter dadurch gekennzeichnet, dass bei Aufnahme des IR-Spektrometers in die Aufnahmevorrichtung eine IR-Strahlung durch die mit mindestens einer Folie abgedeckten Öffnungen derart gerichtet werden kann, dass die IR-Strahlung den Durchgangskanal schneidet.

In manchen Ausführungsformen ist der Adapter dadurch gekennzeichnet, dass mindestens eine Aufnahme-Seitenwand, mindestens eine Aufnahme-Rastung umfasst, wobei die Aufnahme-Rastung eingerichtet und ausgebildet ist, das IR-Spektrometer in einer Messposition zu fixieren.

Die Aufgabe wird ferner gelöst durch ein System zur Analyse eines Atemgasstromes mindestens umfassend ein Beatmungsgerät und/oder ein Patienteninterface und ein IR-Spektrometer wobei das System mindestens einen erfindungsgemäßen Adapter umfasst, wobei der Adapter mit dem Beatmungsgerät und/oder dem Patienteninterface gasleitend verbunden ist und wobei das IR-Spektrometer den Adapter zumindest bereichsweise derart umschließt, dass IR-Strahlung den Atemgasstrom schneidet.

In manchen Ausführungsformen ist das System dadurch gekennzeichnet, dass der Adapter im und/oder am Beatmungsgerät und/oder im und/oder am Patienteninterface angeordnet ist.

In manchen Ausführungsformen ist das System dadurch gekennzeichnet, dass der Adapter über mindestens eine Leitung mit dem Beatmungsgerät und/oder dem Patienteninterface verbunden ist.

In den Figuren sind Ausführungsbeispiele des erfindungsgemäßen Adapters dargestellt. Es zeigen:
Fig. 1 eine Übersicht über einen erfindungsgemäßen Adapter zur Verwendung mit Spektrometern für die Analyse von Gasen, beispielsweise von Atemgasen, in Draufsicht von der Seite
Fig. 2 eine perspektivische Ansicht eines erfindungsgemäßen Adapters
Fig. 3 einen erfindungsgemäßen Adapter in Draufsicht von der Seite
Fig. 4 einen erfindungsgemäßen Adapter in Draufsicht von der Seite, wobei zusätzlich Abdeckungen dargestellt sind (A), die zur Herstellung eines Betriebsmodus auf den Adapter aufgebracht werden (B), sowie ein IR-Spektrometer 80, das sich auf dem betriebsbereiten Adapter 10 in einer Messposition befindet (C)
Fig. 5 eine Folie für einen erfindungsgemäßen Adapter
Fig. 6 einen erfindungsgemäßen Adapter in Draufsicht von oben
Fig. 7 einen erfindungsgemäßen Adapter in Draufsicht von unten
Fig. 8 einen Längsschnitt nach Linie G-G durch einen erfindungsgemäßen Adapter
Fig. 9 einen erfindungsgemäßen Adapter in Draufsicht von der Gehäuseeingangsseite aus
Fig. 10 einen erfindungsgemäßen Adapter in Draufsicht von der Gehäuseausgangsseite aus
Fig. 11 einen Querschnitt nach Linie B-B, der im Wesentlichen durch den Grundkörper eines erfindungsgemäßen Adapters führt
Fig. 12 eine Detailansicht des Schnitts nach Linie B-B im Bereich Y
Fig. 13 eine Detailansicht des Schnitts nach Linie B-B im Bereichs X
Fig. 14 eine schematische Übersicht über ein System mit einem erfindungsgemäßen Adapter mit aufgesetztem IR-Spektrometer, der über eine Leitung mit einem Beatmungsgerät und einem Patienteninterface gasleitend verbunden ist.

### Ausführungsbeispiele

In den nachfolgenden Ausführungsbeispielen ist ein erfindungsgemäßer Adapter 10 gezeigt. Weitere Merkmale und Vorteile der vorliegenden Erfindung werden in den nachfolgenden Beschreibungen von Ausführungsbeispielen anhand der Figuren deutlich. Die Erfindung ist nicht auf die dargestellten Ausführungsbeispiele beschränkt.

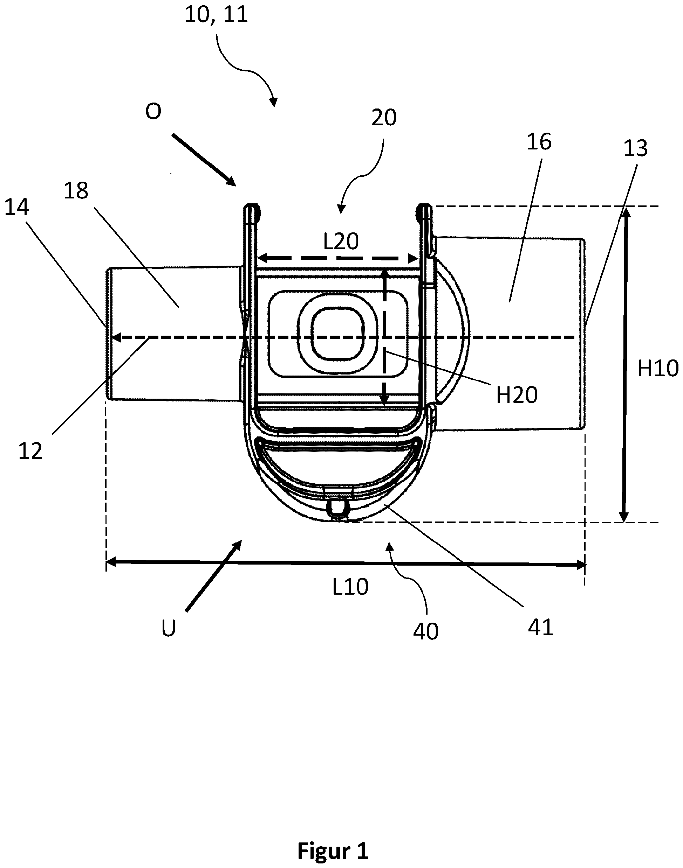

Figur 1 zeigt eine Übersicht über einen erfindungsgemäßen Adapter 10 zur Verwendung mit Spektrometern für die Analyse von Gasen, beispielsweise von Atemgasen in Draufsicht von der Seite. Der Adapter 10 kann auch als Küvette oder Durchflussküvette bezeichnet werden.

Der Adapter 10 ist eingerichtet und ausgebildet, Gase derart aufzunehmen, dass diese analysiert werden können. Für die Analyse wird ein zu dem Adapter 10 formkomplementär ausgebildetes Spektrometer verwendet, beispielsweise ein Infrarot-Spektrometer (IR-Spektrometer) 80 (nicht gezeigt). Mit dem IR-Spektrometer 80 kann beispielsweise der CO₂-Gehalt eines Atemgases bestimmt werden. Andere quantitative oder qualitative Analysen sind ebenfalls denkbar.

Der Adapter 10 ist eingerichtet und ausgebildet, dass eine infrarote Strahlung (IR-Strahlung) derart durch den Adapter 10 hindurchgeführt werden kann, dass die Strahlung das zu analysierende Atemgas im Adapter 10 schneidet.

Der Adapter 10 ist beispielsweise aus einem Kunststoff gefertigt. Der Adapter 10 ist insbesondere aus einem thermoplastischem Kunststoff gefertigt. Der Adapter 10 kann beispielsweise über ein Spritzgussverfahren hergestellt werden. Denkbar sind aber auch andere geeignete Werkstoffe und Herstellungsverfahren, die in Bezug auf Fertigungseigenschaften, Kosten, Gewicht, Schweißbarkeit und Temperaturbeständigkeit geeignet sind.

Geeignete Kunststoffe umfassen Polycarbonate (PC), Polymethylmethacrylate (PMMA), Polystyrole (PS) oder Cycloolefin-Copolymer (COC, Topas). Beispielsweise ist der Adapter 10 ausgewählt aus der Gruppe PMMA Plexiglas 7N, Topas 6017-S04, PC Lexan 121 RM1, PC Makrolon 2400, PS Styrolution 124 L, Topas 8007 oder COC 8007 X-04. Bevorzugt ist der Adapter 10 aus COC 8007-04 hergestellt.

Der Adapter 10 kann zumindest einen Griff 41 aufweisen. Per definitionem ist die Seite, an der sich der Griff 41 befindet, die untere Seite oder unten (U). Die Seite, die dem Griff 41 gegenüberliegt ist per definitionem die obere Seite oder oben (O).

Der Adapter 10 umfasst ein Gehäuse 11 und einen Durchgangskanal 12.

Der Adapter 10 hat eine maximale Länge L10 und eine maximale Höhe H10. Die Länge L10 ist in der Regel größer als die Höhe H10. Länge L10 und Höhe H10 können jedoch auch gleich sein und die Höhe H10 kann auch größer sein als die Länge L10. Der Durchgangskanal 12 erstreckt sich durch die gesamte Länge L10 des Adapters 10.

Der Adapter 10 hat zumindest eine Eintrittsöffnung 13 und eine Austrittsöffnung 14. Der Durchgangskanal 12 erstreckt sich von der Eintrittsöffnung 13 zu der Austrittsöffnung 14. Der Durchgangskanal 12 ist eingerichtet und ausgebildet, Gase, insbesondere Atemgase, zu führen. Das Atemgas kann in den Adapter 10 eingeleitet werden und/oder diesen entlang des Durchgangskanals 12 durchströmen.

Das Gehäuse 11 umfasst in der beispielhaften Ausführungsform im Wesentlichen vier Bauteile, nämlich einen Gehäuseeingang 16, einen Gehäuseausgang 18, eine Aufnahmevorrichtung 40 und einen Grundkörper 20.

Die Aufnahmevorrichtung 40 ist zwischen dem Gehäuseeingang 16 und dem Gehäuseausgang 18 angeordnet. Der Grundkörper 20 ist benachbart zu der Aufnahmevorrichtung 40 angeordnet. Die Aufnahmevorrichtung 40 hat von der Seite aus betrachtet im Wesentlichen eine U-Form. Der Grundkörper 20 befindet sich innerhalb der U-Form. Der Grundkörper 20 wird somit von der Aufnahmevorrichtung 40 U-Förmig umschlossen.

Aufnahmevorrichtung 40 und Grundkörper 20 sind eingerichtet und ausgebildet, ein formkomplementäres IR-Spektrometer 80 derart aufzunehmen, dass zumindest Teile des Grundkörpers 20 vom IR-Spektrometer 80 umschlossen sind.

An dem Gehäuseeingang 16 und Gehäuseausgang 18 können Anschlüsse angeschlossen werden. Beispielsweise können Schläuche angeschlossen werden, die eine Verbindung zu einer Gasquelle und/oder zu einem Patienteninterface ausbildet (nicht gezeigt). Das Atemgas kann somit über den Gehäuseeingang 16 in den Adapter 10 eingeleitet werden und über den Gehäuseausgang 18 aus dem Adapter 10 herausgeführt werden und vice versa. Das Atemgas befindet sich sodann im und/oder durchläuft sodann den Durchgangskanal 12.

Vorteilhafterweise weist der Adapter 10 eine kompakte Bauweise auf. Dadurch kann das Volumen des Durchgangskanals 12 möglichst gering gehalten werden.

Die gesamte Länge L10 des Adapters 10 beträgt maximal 120 mm, bevorzugt weniger als 80 mm. In einer beispielhaften Ausführungsform liegt die Länge L10 des Adapters 10 in einem Bereich von 70 mm bis 30 mm. Die Länge L10 des Adapters 10 beträgt beispielsweise 55,6 mm.

In manchen Ausführungsformen können beispielsweise Gehäuseeingang 16 und Gehäuseausgang 18 kleinere Abmessungen aufweisen, wodurch die gesamte Länge L10 des Adapters 10 reduziert werden kann. Gehäuseeingang 16 und Gehäuseausgang 18 können in anderen Ausführungsformen auch länger ausgebildet sein, so dass die gesamte Länge L10 des Adapters zunimmt. Die Länge des Durchgangskanals 12 steht in direkter Abhängigkeit zur Länge L10.

Die Höhe H10 des Adapters 10 beträgt maximal 120 mm, bevorzugt weniger als 80 mm. In einer beispielhaften Ausführungsform liegt die Höhe H10 des Adapters 10 in einem Bereich von zwischen 60 mm bis 20 mm. Die Höhe H10 des Adapters 10 beträgt beispielsweise 36,75 mm.

Die Höhe H10 des Adapters 10 richtet sich in der vorliegenden Ausführungsform nach den Abmessungen der Aufnahmevorrichtung 40 und des Griffes 41. Beispielsweise kann eine alternative Ausführungsform des Griffes 41 die Gesamthöhe H10 des Adapters 10 vergrößern oder verkleinern. Eine alternative Ausgestaltung der Aufnahmevorrichtung 40 kann die Gesamthöhe H10 des Adapters ebenfalls vergrößern oder verkleinern. Der Durchgangskanal 12 steht nicht in direkter Abhängigkeit zur Höhe H10 des Adapters 10 in der vorliegenden Ausführungsform.

Das Volumen des Durchgangskanals steht in Abhängigkeit von der Länge L10 des Adapters 10. In der vorliegenden Ausführungsform fasst der Durchgangskanal 12 ein Volumen von 6,6 ml. Ideal ist ein geringst möglichstes Volumen zwischen 0,5 und 7 ml je nach Patientenkategorie.

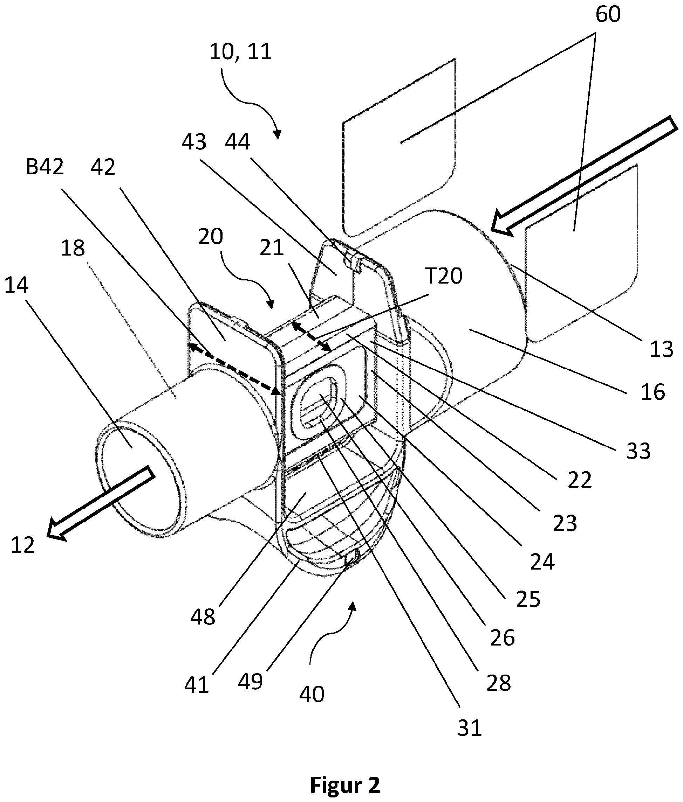

Der Grundkörper 20 ist ein weiteres der vier wesentlichen Bauteile des Adapters 10. Der Grundkörper 20 hat eine Höhe H20, eine Länge L20 und eine Tiefe T20 (siehe Fig. 1 und 2). Im vorliegenden Ausführungsbeispiel ist die Länge L20 größer als die Höhe H20. Länge L20 und Höhe H20 können auch gleich ausgebildet sein und die Höhe H20 kann in anderen Ausführungsbeispielen auch größer als die Länge L20 sein.

In dem Grundkörper 20 findet die Gas-Analyse statt. Zu diesem Zweck sollte der Grundkörper 20 ein möglichst kleines Volumen aufweisen, aber gleichzeitig keinen nennenswerten Strömungswiderstand darstellen. Die Abmessungen des Grundkörpers 20 können je nach Art und Anzahl der Messungen optimiert werden.

Die Länge L20 des Grundkörpers 20 beträgt zwischen 60 und 4 mm, bevorzugt zwischen 30 und 10 mm, noch stärker bevorzugt zwischen 21 und 15 mm. Die Länge L20 des Grundkörpers 20 beträgt beispielsweise zwischen 19,3 und 19,5 mm. Die Höhe H20 des Grundkörpers 20 beträgt zwischen 60 und 4 mm, bevorzugt zwischen 30 und 10 mm, noch stärker bevorzugt zwischen 16 und 13 mm. Die Höhe H20 des Grundkörpers 20 beträgt beispielsweise zwischen 15 und 15,2 mm. Die Tiefe T20 des Grundkörpers 20 beträgt zwischen 60 und 2 mm, bevorzugt zwischen 20 und 4 mm. Die Tiefe T20 des Grundkörpers 20 beträgt beispielsweise 7,55 mm (siehe Figur 2).

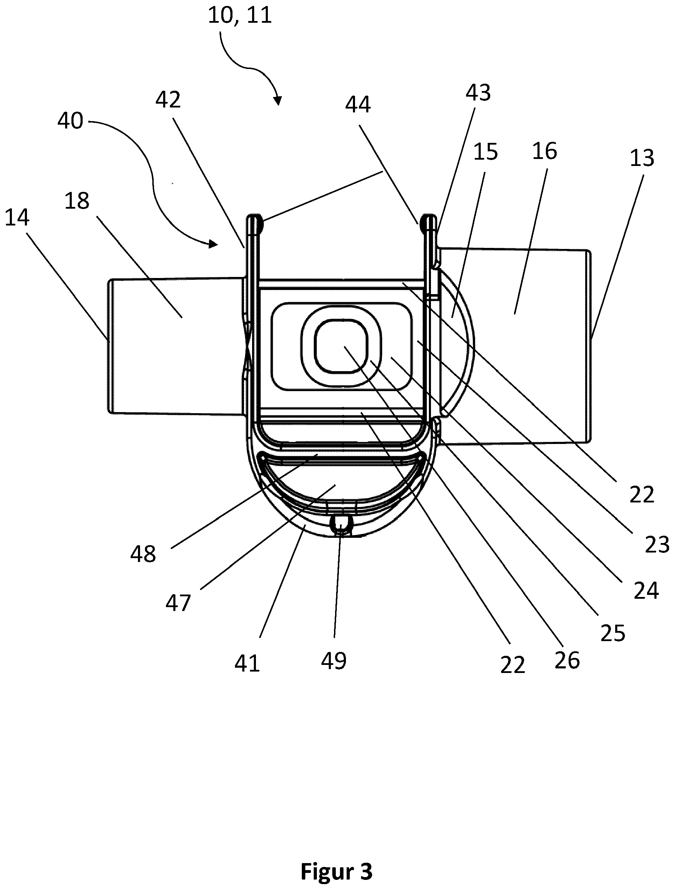

Figur 2 zeigt eine perspektivische Ansicht eines erfindungsgemäßen Adapters 10. Figur 3 zeigt einen erfindungsgemäßen Adapter 10 in Draufsicht von der Seite.

In Figur 2 ist das Gehäuse 11 mit den vier Bauteilen Gehäuseeingang 16, Gehäuseausgang 18, Aufnahmevorrichtung 40 und Grundkörper 20 in einer erfindungsgemäßen beispielhaften Ausführungsform abgebildet. Der Gehäuseeingang 16 und der Gehäuseausgang 18 sind in Form eines länglichen Hohlkörpers ausgebildet. Gehäuseeingang 16 und Gehäuseausgang 18 weisen in der Regel einen kreisrunden Querschnitt auf. Eine ovale oder eckige Form ist auch möglich. Gehäuseeingang 16 und Gehäuseausgang 18 sind beispielsweise in Form eines länglichen Hohlkörpers mit einem kreisrunden Querschnitt ausgebildet. Gehäuseeingang 16 und Gehäuseausgang 18 können auch als Anschlussstutzen bezeichnet werden. Der Gehäuseeingang 16 weist im vorliegenden Ausführungsbeispiel einen größeren Durchmesser auf als der Gehäuseausgang 18. Es ist auch möglich, dass Gehäuseeingang 16 und Gehäuseausgang 18 einen gleichen Durchmesser aufweisen und dass der Durchmesser des Gehäuseausgangs 18 größer ist als der des Gehäuseeingangs 16.

Der Gehäuseeingang 16 umfasst die Eintrittsöffnung 13. Durch die Eintrittsöffnung 13 können Gase in den Adapter 10 eingeleitet werden. Die Eintrittsöffnung 13 stellt den Beginn des Durchgangskanals 12 dar. Die Gase gelangen somit durch die Eintrittsöffnung 13 in den Durchgangskanal 12.

Der Gehäuseausgang 18 umfasst die Austrittsöffnung 14. Durch die Austrittsöffnung 14 können Gase aus dem Adapter 10 herausgeleitet werden. Die Austrittsöffnung 14 stellt das Ende des Durchgangskanals 12 dar. Die Gase werden somit in der Regel durch die Eintrittsöffnung 13 in den Durchgangskanal 12 geleitet und werden durch die Austrittsöffnung 14 aus dem Durchgangskanal 12 herausgeleitet. Ein umgekehrter Gasfluss ist auch möglich.

Zwischen dem Gehäuseeingang 16 und dem Gehäuseausgang 18 befindet sich die Aufnahmevorrichtung 40 und der Grundkörper 20.

Der Grundkörper 20 ist in Form eines Hohlkörpers ausgebildet. Der Grundkörper 20 ist beispielsweise als Vierkantrohr mit vier planparallelen Flächen ausgebildet. Die vier Flächen des Grundkörper 20 stehen bevorzugt im Wesentlichen rechtwinklig zueinander. Der Durchgangskanal 12 führt durch den Grundkörper 20 hindurch.

Der Grundkörper 20 umfasst eine Oberseite 21, eine Unterseite 31 und zwei Seitenwände 33. Die Oberseite 21 bzw. die Unterseite 31 definieren eine Tiefe des Grundkörpers T20. Die Seitenwände 33 definieren eine Höhe des Grundkörpers H20 und eine Länge des Grundkörpers L20 (siehe Fig. 1).

Oberseite 21 und Unterseite 31 sind parallel zueinander. Oberseite 21 und Unterseite 31 sind beispielsweise eben und geschlossen ausgebildet. Die Kanten von Oberseite 21 und Unterseite 31 zu den Seitenwänden 33 können scharfkantig sein. Im gezeigten Ausführungsbeispiel sind die scharfen Kanten durch eine Fase 21 abgeschrägt.

Die Seitenwände 33 sind parallel zueinander. In Fig. 2 ist lediglich eine Seitenwand 33 abgebildet, die entsprechende zweite Seitenwand 33 ist im vorliegenden Ausführungsbeispiel spiegelsymmetrisch ausgebildet. Unterschiedlich ausgebildete Seitenwände 33 sind auch denkbar. Unterschiedlich ausgebildete Seitenwände 33 sind beispielsweise denkbar, um ein IR-Spektrometer 80 in einer vorbestimmten Orientierung aufzunehmen und somit eine Messrichtung vorzugeben.

Die Seitenwände 33 umfassen jeweils mindestens eine Öffnung 26,27 mit je einer Laibung 28. Mehrere Öffnungen sind auch denkbar, um beispielsweise verschiedene Messungen parallel durchführen zu können. Im vorliegenden Ausführungsbeispiel weist der Adapter 10 zwei Öffnungen 26, 27 auf, von denen in den Figuren 2 und 3 nur eine abgebildet ist. Die Öffnungen 26, 27 sind identisch ausgebildet. Die Öffnungen 26, 27 sind in ihrer Grundform ein Polygon. Die Öffnungen 26, 27 bilden in diesem Ausführungsbeispiel ein Viereck. Die Ecken des Polygons können eckig oder abgerundet sein. In diesem Ausführungsbeispiel sind die Öffnungen 26, 27 beispielsweise in Form eines Quadrates mit abgerundeten Ecken ausgebildet.

Jede Öffnung weist jeweils eine Laibung 28 auf. Die Laibung 28 steht senkrecht zu der Seitenwand 33. Die Laibung 28 ist die innere, der Öffnung 26, 27 zugewandte Wandfläche der Seitenwand 33. Die Laibung 28 umfasst die Öffnung 26, 27 vollständig. Die Laibung 28 weist bevorzugt eine konstante Tiefe auf.

In einer einfachen Ausführungsform enthalten die Seitenwände 33 nur die Öffnungen 26, 27 und die Laibung 28. Dann entspricht die Tiefe der Laibung 28 der Dicke der Seitenwand 33. In der in den Figuren dargestellten Ausführungsform umfassen die Seitenwände 33 neben der Öffnung 26, 27 und der Laibung 28 zusätzlich je einen inneren Rand 25, eine Seitenfläche 23 und einen äußeren Rand 24.

Der innere Rand 25 umgibt die Öffnung 26, 27 vollständig. Der innere Rand 25 kann jegliche geeignete Form aufweisen. Der innere Rand 25 kann beispielsweise eine der Öffnung 26, 27 entsprechende Form aufweisen. Der innere Rand 25 ist beispielsweise in Form eines quadratischen Rahmens mit abgerundeten Ecken ausgebildet. Die Tiefe der Laibung 28 entspricht in diesem Beispiel der Dicke des inneren Randes 25. Die Laibung 28 ist zwischen 0,4 mm und 4 mm tief. Die Laibung 28 ist beispielsweise 0,9 mm tief. Die Laibung 28 sollte eine möglichst geringe Tiefe aufweisen, um Verwirbelungen des Gasflusses im Durchgangskanal 12 zu vermeiden und Messfehler zu minimieren.

Der äußere Rand 23 begrenzt die Seitenwand 33 nach außen hin. Der äußere Rand 23 hat eine rechteckige Form. Der äußere Rand 23 ist entsprechend der Form der Seitenwand 33 ausgebildet. Im vorliegenden Ausführungsbeispiel ist der äußere Rand 23 dementsprechend in seiner Breite größer als in seiner Höhe. Der innere Rand 25 und der äußere Rand 23 haben im vorliegenden Ausführungsbeispiel ein identisches Höhenprofil.

Im vorliegenden Ausführungsbeispiel ist zwischen dem inneren Rand 25 und dem äußeren Rand 23 eine Seitenfläche 24 angeordnet. Die Seitenfläche ist im Vergleich zum inneren Rand 25 und zum äußeren Rand 23 leicht vertieft. Die Vertiefung beträgt von 0,1 mm bis 3 mm, beispielsweise 0,5 mm. Die Seitenfläche 24 ist im vorliegenden Ausführungsbeispiel rechteckig und in seiner Breite größer als in seiner Höhe.

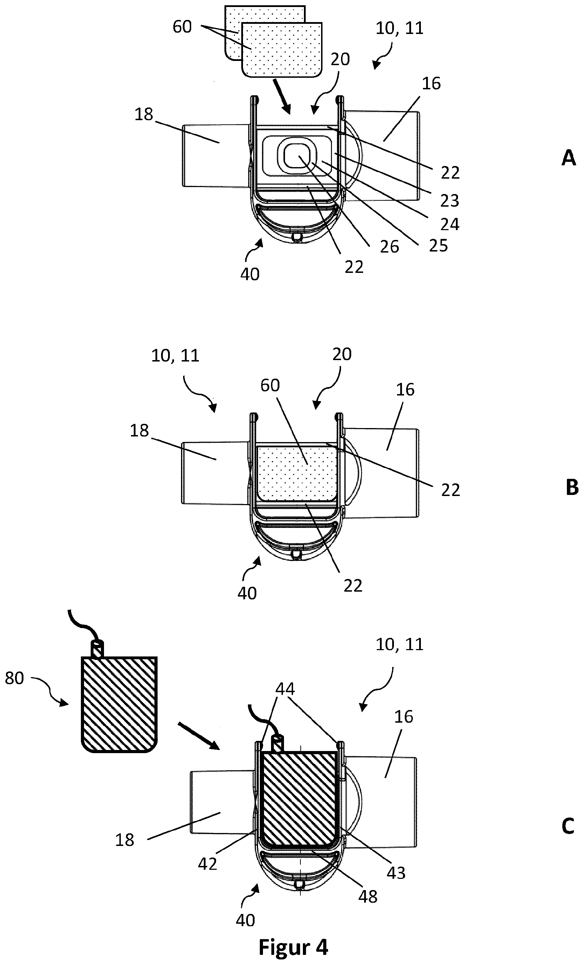

Durch die spiegelsymmetrische Ausbildung der Seitenflächen 23 befinden sich die Öffnungen 26 und 27 an einer sich entsprechenden Position innerhalb der Seitenflächen 23. Die Öffnungen 26, 27 sind somit beidseitig des Durchgangskanals 12 an sich gegenüberliegen Seiten angeordnet. Bei Aufnahme eines formkomplementären IR-Spektrometers 80 wird der Grundkörper 20 in einer Weise vom IR-Spektrometer 80 umschlossen (siehe Fig. 4C), dass IR-Strahlung derart durch die Öffnungen 26, 27 gerichtet werden kann, dass die IR-Strahlung das im Grundkörper 20 befindliche Atemgas schneidet.

Das vierte Bauteil des Adapters 10 ist die Aufnahmevorrichtung 40. Die Aufnahmevorrichtung 40 ist eingerichtet und ausgebildet, ein formkomplementäres IR-Spektrometer 80 aufzunehmen und in einer Messposition zu fixieren.

Die Aufnahmevorrichtung 40 hat von der Seite aus betrachtet im Wesentlichen eine U-Form (siehe Figur 3). Die Aufnahmevorrichtung 40 ist benachbart zu dem Grundkörper 20 angeordnet. Der Grundkörper 20 wird von der Aufnahmevorrichtung 40 U-Förmig umschlossen.

Die Aufnahmevorrichtung 40 umfasst zwei Aufnahme-Seitenwände 42, 43 mit mindestens einer, bevorzugt zwei Aufnahme-Rastungen 44 und einen Aufnahme-Boden 48.

Die erste Aufnahme-Seitenwand 42 ist benachbart zum Grundkörper 20 und zum Gehäuseausgang 18 angeordnet. Die zweite Aufnahme-Seitenwand 43 ist benachbart zum Grundkörper 20 und zum Gehäuseeingang 16 angeordnet. Die Aufnahme-Seitenwände 42, 43 sind senkrecht zu den Seitenwänden 33 und der Oberseite 21 bzw. der Unterseite 31 des Grundkörpers 20 angeordnet. Die Aufnahme-Seitenwände 42,43 sind sattelartig auf dem Adapter 10 angeordnet.

Die Aufnahme-Seitenwände 42,43 sind größer als der Grundkörper 20 ausgebildet. Die Aufnahme-Seitenwände 42, 43 sind breiter ausgebildet als der Grundkörper 20 tief ist. Die Aufnahme-Seitenwände 42, 43 sind höher ausgebildet als der Grundkörper 20 hoch ist. Die Aufnahme-Seitenwände 42, 43 ragen somit über die Tiefe des Grundkörpers T20 und über die Höhe des Grundkörpers H20 hinaus.

Am unteren Ende sind die Aufnahme-Seitenwände 42, 43 über einen Aufnahme-Boden 48 miteinander verbunden. Die Aufnahme-Seitenwände 42, 43 stehen beispielsweise senkrecht auf dem Aufnahme-Boden 48. Es ist auch denkbar, dass die Aufnahme-Seitenwände 42,43 nach außen geneigt auf dem Aufnahme-Boden 48 angeordnet sind. Am oberen Ende sind die Aufnahme-Seitenwände 42, 43 nicht verbunden. Dadurch ergibt sich eine U-Form.

Der Übergang von den Aufnahme-Seitenwänden 42, 43 zum Aufnahme-Boden 48 kann eckig sein. Der Übergang kann wie in den Figuren 2 und 3 dargestellt auch abgerundete Ecken aufweisen. Der Aufnahme-Boden 48 ist beispielsweise eben ausgebildet. Der Aufnahme-Boden 48 kann auch gerundet ausgebildet sein. Der Aufnahme-Boden 48 ist breiter ausgebildet als der Grundkörper 20 tief ist. Der Aufnahme-Boden 48 ist somit auch breiter ausgebildet als die Unterseite 31. Der Aufnahme-Boden 48 ist beispielsweise ebenso breit ausgebildet wie die Aufnahme-Seitenwände 42, 43 (siehe Figur 2).

Dadurch, dass sowohl die Aufnahme-Seitenwände 42, 43 als auch der Aufnahme-Boden 48 breiter ausgebildet sind als der Grundkörper 20 tief ist, werden freie Flächen bereitgestellt, an denen ein formkomplementär ausgebildetes IR-Spektrometer 80 anliegen kann (siehe weiter unten hierin, Fig. 4C). Die Seitenwände 42, 43 und der Aufnahme-Boden 48 bieten zudem eine Verschattung des Grundkörpers, was bei einer Messung mit dem IR-Spektrometer 80 von Vorteil ist. Die AufnahmeVorrichtung 40 schirmt die umgebenden Strahlung ab. So werden bei einer spektrometrischen Messung Messfehler durch den Einfluss von Umgebungsstrahlung vermindert oder vermieden.

Der Adapter 10 kann einen Griff 41 umfassen. Der Griff 41 ist unter dem Aufnahme-Boden 48 angeordnet. Der Griff 41 kann jegliche geeignete Form aufweisen. Der Griff 41 weist im vorliegenden Ausführungsbeispiel eine halbkreisförmige Form.

Bei einer Herstellung des Adapters 10 über ein Spritzgussverfahren kann sich innerhalb des Griffes 41 ein Anspritzpunkt 49 befinden.

Figur 4 zeigt einen erfindungsgemäßen Adapter 10 in Draufsicht von der Seite, wobei zusätzlich Abdeckungen 60 dargestellt sind (A), die zur Herstellung eines Betriebsmodus auf den Adapter 10 aufgebracht werden (B), sowie ein IR-Spektrometer 80, das sich auf dem betriebsbereiten Adapter 10 in einer Messposition befindet (C).

Figur 4A zeigt einen erfindungsgemäßen Adapter 10, der sich nicht in einem Betriebsmodus befindet, da die Abdeckung 60 noch nicht aufgebracht wurde. In diesem Falle sind alle Öffnungen 26,27 offen. Zur Herstellung eines Betriebsmodus des Adapters 10 wird mindestens eine Abdeckung 60 auf den Adapter 10 aufgebracht.

In Figur 4B ist ein Adapter von der Seite gezeigt und somit ist lediglich eine Seitenwand 33 mit einer abgedeckten Öffnung 26 dargestellt. Die nicht abgebildete Öffnung 27 auf der anderen Seitenwand 33 kann in diesem Beispiel mit einer zweiten Abdeckung 60 abgedeckt werden (nicht gezeigt).

Der erfindungsgemäße Adapter 10 umfasst somit neben den vier oben beschriebenen Bauteilen Gehäuseeingang 16, Gehäuseausgang 18, Aufnahmevorrichtung 40 und Grundkörper 20 mindestens eine Abdeckung 60.

Mit der Abdeckung 60 wird zur Herstellung eines Betriebsmodus mindestens eine der mindestens zwei Öffnungen 26, 27 abgedeckt. In manchen Ausführungsformen ist es denkbar, dass nur eine der mindestens zwei Öffnungen 26 ,27 zur Herstellung eines Betriebsmodus abgedeckt wird. In diesem Falle sollte diejenige Öffnung 26, 27 abgedeckt werden, die sich zwischen einem Sensor des IR-Spektrometers 80 und dem Adapter 10 befindet. Die Öffnung 26 27, die sich zwischen einem Empfänger des IR-Spektrometers 80 und dem Adapter 80 befindet kann in manchen Ausführungsformen geöffnet bleiben. Vorteilhafter Weise und in den folgenden Ausführungsbeispielen dargestellt, werden zur Herstellung eines Betriebsmodus beide der mindestens zwei Öffnungen 26, 27 abgedeckt. Die Öffnungen 26, 27 können sodann mit einer identisch ausgebildeten Abdeckung 60 abgedeckt werden. Es ist jedoch auch denkbar, dass die unterschiedlichen Öffnungen 26, 27 mit unterschiedlich ausgebildeten Abdeckungen 60 abgedeckt werden, die sich in der Wahl des Materials, in ihrer Form, Dicke oder anderen Eigenschaften voneinander unterscheiden.

Adapter 10 und Abdeckung 60 können einstückig oder zweistückig ausgeführt sein. Eine zweistückige Ausführung bietet den Vorteil einer einfachen und kostengünstigen Herstellung. Adapter 10 und Abdeckung 60 können aus demselben Material hergestellt sein oder aus unterschiedlichen Materialien gefertigt sein.

Die Abdeckung 60 muss aus einem Material gefertigt werden, das Strahlung durchlässt. Die Abdeckung 60 muss insbesondere für IR-Strahlung durchlässig sein. Die Abdeckung 60 sollte eine Durchlässigkeit für Infrarotstrahlung von mindestens 0,5 aufweisen. Das bedeutet, dass mindestens 50 % der Infrarot-Strahlung durch die Abdeckung 60 hindurch gelassen werden muss, um eine sichere und reproduzierbare Messung zu ermöglichen.

Die Abdeckung 60 kann eine Folie 60 sein. Die Folie 60 kann beispielsweise aus einem Kunststoff hergestellt sein. Geeignete Kunststoffe umfassen Polycarbonate (PC), Polymethylmethacrylate (PMMA), Polystyrole (PS) oder Cycloolefin-Copolymer (COC, Topas). Beispielsweise ist die die Folie 60 aus PMMA Plexiglas 7N, Topas 6017-S04, PC Lexan 121 RM1, PC Makrolon 2400, PS Styrolution 124 L, Topas 8007 oder COC 8007 X-04. Bevorzugt ist die Folie 60 aus COC 8007 X-04 hergestellt.

Im vorliegenden Ausführungsbeispiel sind Adapter 10 und Folie 60 zweistückig ausgeführt und aus dem gleichen Materialien gefertigt. Die Wahl der Materialien von Adapter 10 und Folie 60 kann auch unabhängig voneinander ausgewählt sein.

Die Folie 60 weist eine geeignete Dicke auf, die IR-Strahlung in ausreichendem Maße durchlässt, um präzise und reproduzierbare Messungen zu ermöglichen. Die Folie 60 ist zwischen 2000 µm und 50 µm dick, bevorzugt zwischen 1000 µm und 100 µm, besonders bevorzugt zwischen 600 µm und 100 µm, beispielsweise 140 µm dick.

Zur Herstellung eines Betriebsmodus des Adapters 10 wird die mindestens eine Folie 60 auf den Adapter 10 aufgebracht (siehe Fig. 4B). Die Aufbringung der Folie 60 auf den Adapter 10 kann reversibel oder irreversibel sein. Die Folie 60 ist größer ausgebildet als die Öffnung 26, 27. Die Folie 60 deckt die Öffnungen 26, 27 im Betriebsmodus vollständig ab. Zu diesem Zwecke wird die Folie 60 mindestens bereichsweise mit mindestens einer Seitenwand 33 des Grundkörpers 20 verbunden. Die Folie 60 kann mit der gesamten Seitenwand 33 verbunden werden oder mit Teilen der Seitenwand 33. Die Folie 60 kann beispielsweise mit dem inneren Rand 25 und/oder dem äußeren Rand 24 und/oder der Seitenfläche 23 verbunden werden.

Im vorliegenden Ausführungsbeispiel wird pro Öffnung 26 oder 27 je eine Folie 60 verwendet. Die Folie 60 ist beispielsweise so groß ausgebildet, dass sie nahezu eine gesamte Seitenwand 33 abdeckt. Die Folie 60 deckt in diesem Beispiel die Öffnung 26, 27, den inneren Rand 25, den äußeren Rand 24 und die Seitenfläche 23 ab. Lediglich die Fasen 21 werden nicht von der Folie abgedeckt (siehe Fig. 4 B).

Es ist auch denkbar, dass die Folie 60 kleiner ausgebildet ist und beispielsweise lediglich die Öffnungen 26, 27 und den inneren Rand 25 abdecken. Die Folie 60 kann jede geeignete Größe aufweisen. Es ist auch denkbar, dass die Folie 60 größer ausgebildet ist als in Figur 4 gezeigt. Beispielsweise ist denkbar, dass eine einzige Folie 60 beide Öffnungen 26, 27 gleichzeitig abdecken kann. In einer derartigen Ausführungsform kann die Folie 60 von einer Seitenwand 33 über die Oberseite 21 und/oder über die Unterseite 31 zu der zweiten Seitenwand 33 verlaufen (nicht gezeigt).

Figur 4C zeigt schematisch einen Adapter 10 im Betriebsmodus, auf den ein IR-Spektrometer 80 aufgesetzt wurde. Der IR-Spektrometer 80 wird derart von oben auf den betriebsbereiten Adapter 10 aufgesetzt, dass es mindestens die Öffnungen 26 ,27 des Grundkörpers umgibt.

Das IR-Spektrometer 80 umfasst ein Mittel zum Aussenden von IR-Strahlung, auch Sensor genannt, ein Mittel zum Empfangen von IR-Strahlung, auch Empfänger genannt, eine Analyseeinrichtung der empfangenen Strahlung sowie optional ein Kabel zur Datenübertragung (nicht gezeigt).

Die Form der Aufnahme-Seitenwände 42, 43 und des Aufnahme-Bodens 48 ist formkomplementär zu einem kompatiblen IR-Spektrometer 80 ausgebildet. Das IR-Spektrometer 80 wird in einer Weise über den Adapter 10 gestülpt, dass es zwischen den Seitenwänden 42, 43 und dem Aufnahme-Boden 48 angeordnet ist und den Grundkörper 20 im Wesentlichen umschließt.

Die mindestens eine Aufnahme-Rastung 44 ist eingerichtet und ausgebildet, das IR-Spektrometer 80 auf dem Adapter 10 einzurasten. Die Aufnahme-Rastung 44 befindet sich am oberen Ende der Aufnahme-Seitenwände 42 und/oder 43. Die Aufnahme-Rastung 44 ist als Vorsprung ausgebildet, der nach innen in die U-förmige Aufnahmevorrichtung 40 hineinragt. Die Aufnahme-Rastung 44 ist somit stets zum Grundkörper 20 hin orientiert. Die Aufnahme-Rastung 44 ist beispielsweise halbkreisförmig auf den Aufnahme-Seitenwänden 42,43 angeformt. Die Aufnahme-Rastung 44 ist beispielsweise 0,5 mm tief ausgebildet.

Die Aufnahme-Rastung 44 kann sich über die gesamte Breite einer Aufnahme-Seitenwand 42, 43 erstrecken. Zur Materialersparnis ist es von Vorteil, die Aufnahme-Rastung 44 möglichst klein zu halten. Die Aufnahme-Rastung 44 kann sich beispielsweise nicht über das gesamte obere Ende der Aufnahme-Seitenwand 42, 43 erstrecken. Die Aufnahme-Rastung 44 ist beispielsweise in der Mitte des oberen Endes der Seitenwand 42, 43 angeordnet.

Das Material der Aufnahme-Seitenwände 42,43 ist leicht nachgiebig ausgebildet. Dadurch kann das IR-Spektrometer 80 mit leichtem Druck von oben auf den Adapter 10 aufgeschoben werden. Die Aufnahme-Seitenwände 42,43 geben unter leichtem Druck etwas nach und erlauben die Aufnahme des IR-Spektrometers 80 in die Aufnahmevorrichtung 40. Wenn sich das IR-Spektrometer 80 in einer Messposition befindet, federn die Aufnahme-Seitenwände 42 43 leicht zurück in ihre Ausgangsposition.

Die Aufnahme-Rastung 44 kann sodann das IR-Spektrometer 80 nach oben hin abgrenzen. Auf diese Weise kann das IR-Spektrometer 80 in seiner Messposition gehalten werden (siehe Fig. 4C). Es ist auch denkbar, dass das IR-Spektrometer 80 ein formkomplementäres Gegenstück zur Aufnahme-Rastung 44 aufweist, in die die Aufnahme-Rastung 44 eingeführt wird.

Die Aufnahme-Rastung 44 ist eingerichtet und ausgebildet, dass ein Anwender, der das IR-Spektrometer 80 in die Aufnahmevorrichtung 40 des Adapters 10 hineinschiebt, eine taktile und/oder akustische Rückmeldung über ein korrektes Einrasten erhält. Nach einem korrekten Einrasten befindet sich das IR-Spektrometer 80 derart auf dem Adapter 10, dass eine Messung mit dem IR-Spektrometer 80 durchgeführt werden kann.

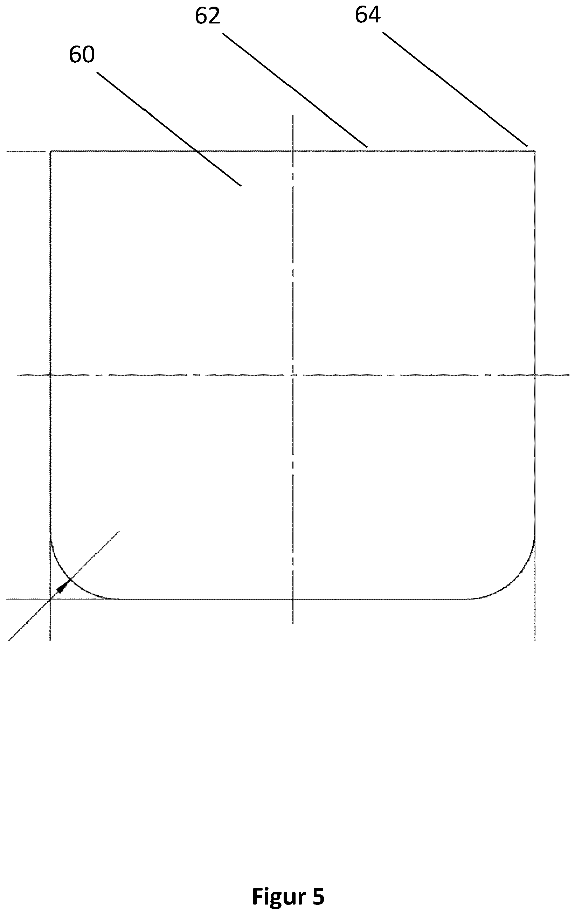

**Figur 5** zeigt eine Folie 60 für den erfindungsgemäßen Adapter 10. Die Folie 60 weist eine polygone Form auf. Beispielsweise weist die Folie 60 eine viereckige Form auf. Es ist auch denkbar, dass die Folie 60 mehrere Ecken aufweist als vier.

Die Folie 60 ist beispielsweise viereckig mit vier Seitenlängen 62 und vier Ecken 64. Die Seitenlängen 62 können gleich lang sein, so dass die Folie 60 quadratisch ist. Die Seitenlängen 62 können auch unterschiedlich lang sein, so dass die Folie 60 rechteckig ist. Die Seitenlängen 62 der Folie 60 weisen eine Länge von 5 mm bis 60 mm auf. Die Seitenlängen 62 der Folie 60 weisen beispielsweise eine Länge von 10 mm bis 30 mm auf. Beispielsweise sind die Seitenlängen 62 zwischen 15 und 20 mm lang. Die Seitenlängen 62 der Folie 60 sind gerade oder gebogen ausgebildet. Beispielsweise sind die Seitenlängen 62 der Folie 60 gerade ausgebildet. Mindestens eine Ecke 64 kann abgerundet sein. Beispielsweise sind zwei Ecken 64 abgerundet. Es ist auch denkbar, dass drei oder mehr Ecken 64 abgerundet sind.

Die Folie 60 kann die Öffnungen 26, 27 derart abdecken, dass der Durchgangskanal 12 luftdicht abgeschlossen ist. Sobald mindestens eine der beiden Öffnungen 26, 27 des Adapters 10 verschlossen sind, befindet sich der Adapter 10 in einem Betriebsmodus. Bevorzugt werden zur Herstellung eines Betriebsmodus beide Öffnungen 26, 27 abgedeckt (siehe Figur 4B).

Die Aufbringung der Folie 60 auf den Adapter 10 kann reversibel oder irreversibel sein. Adapter 10 und Folie 60 können als Einweg oder als Mehrwegprodukte konzipiert sein. In manchen Ausführungsformen können Adapter 10 und Folie 60 als Einwegartikel ausgebildet sein.

In alternativen Ausführungsformen ist es denkbar, dass Adapter 10 und/oder Folie 60 wiederverwendbar sind. In einem vorteilhaften Ausführungsbeispiel könnte der Adapter 10 aus einem sterilisierbarem Material gefertigt sein, das einer Aufbereitung standhält. Die Folie 60 kann für den Reinigungsprozess entfernt werden und für neuerliche Verwendungen des Adapters 10 jeweils erneuert werden. Somit bietet der erfindungsgemäße Adapter 10 den Vorteil, dass er kostengünstig hergestellt werden kann und dennoch - zumindest teilweise - wiederverwendet werden kann.

Die Folie 60 deckt in manchen Ausführungsformen die Öffnungen 26, 27 derart ab, dass ein Durchgang von Gasen ausschließlich von dem Gehäuseeingang 16 zu dem Gehäuseausgang 18 möglich ist und vice versa. Zu diesem Zwecke wird die Folie 60 zumindest bereichsweise mit der Seitenwand 33 oder mit Teilen der Seitenwand 33 des Grundkörpers 20 verklebt, verschweißt und/oder mechanisch verrastet verbunden. Eine Verschweißung kann über die Methode des Laserschweißens vollzogen werden.

Es werden 20 % bis 80 % der Folie 60 mit der Seitenwand 33 oder mit Teilen der Seitenwand 33 verbunden. Bevorzugt werden 30 % bis 60 % der Folie 60 mit der Seitenwand 33 oder mit Teilen der Seitenwand 33 verbunden, beispielsweise 40 % bis 50 %.

Beispielsweise wird die Folie mit dem inneren Rand 25 und/oder dem äußeren Rand 24 und/oder der Seitenfläche 23 verbunden.

Beispielsweise wird die Folie 60 mit dem inneren Rand 25 und/oder dem äußeren Rand 24 und/oder der Seitenfläche 23 über Laserschweißen verbunden.

In einer bevorzugten Ausführungsform werden 40 % bis 50 % der Folie 60 mit dem inneren Rand 25 und dem äußeren Rand 24 über den Prozess des Laserschweißens miteinander verschweißt. Derart kann eine optimale Fixierung und Stabilisierung der Folie 60 erreicht werden.

Die Methode des Laserschweißens bietet den Vorteil, dass die Folie 60 dauerhaft und dicht mit dem Adapter 10 verbunden wird. Folie 60 und Adapter 10 bleiben nach einer Laserverschweißung auch unter erhöhtem Druck fest miteinander verbunden. Die Verschweißung ist besonders dauerhaft und stabil, wenn Adapter 10 und Folie 60 aus dem gleichen Material bestehen. Es ist jedoch auch denkbar, dass Adapter 10 und Folie 60 aus unterschiedlichen Materialien bestehen und dennoch über Laserschweißen miteinander verbunden werden. In dem Falle wäre der Einsatz eines Absorbers in mindestens einem der beiden Fügepartner erforderlich. Der Prozess des Laserschweißens verläuft schnell, einfach und effizient. Das Laserschweißen ermöglicht es, den Adapter 10 in einem einfachen, qualifizierten und validierten Prozess in einen Betriebsmodus zu überführen.

Die Folie 60 kann in die erforderliche oder gewünschte Form geschnitten und/oder gestanzt werden. Diese Art der Herstellung ist besonders effektiv und kostengünstig. Geringe Abweichungen in der Größe der Folie 60 haben keinen wesentlichen Einfluss auf die Qualität des Adapters 10 im Betriebsmodus. Somit kann die Herstellung des betriebsbereiten Adapters 10 einfach und kostengünstig bereitgestellt werden, ohne dass nennenswerte Ausschussware anfällt.

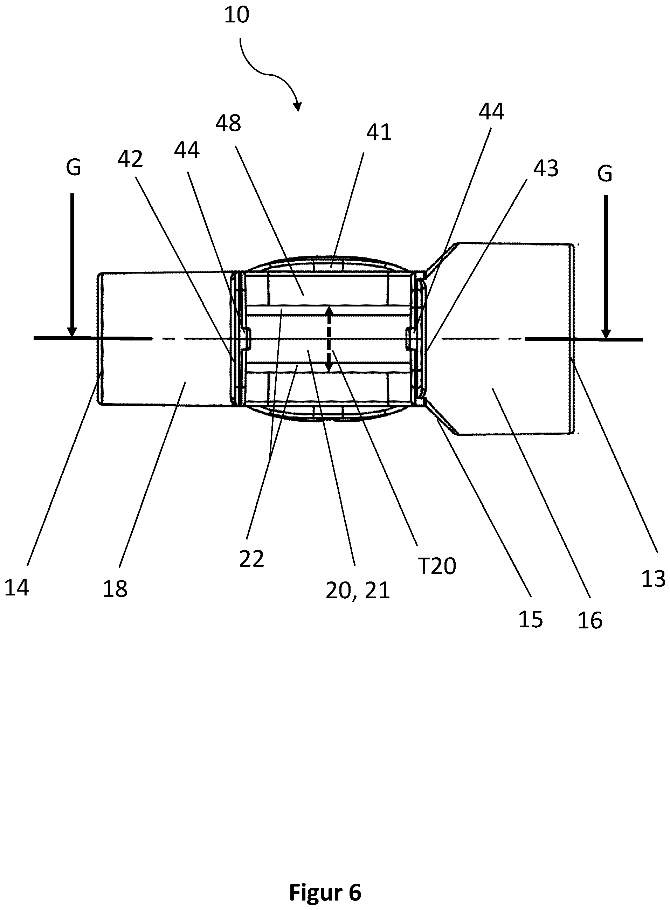

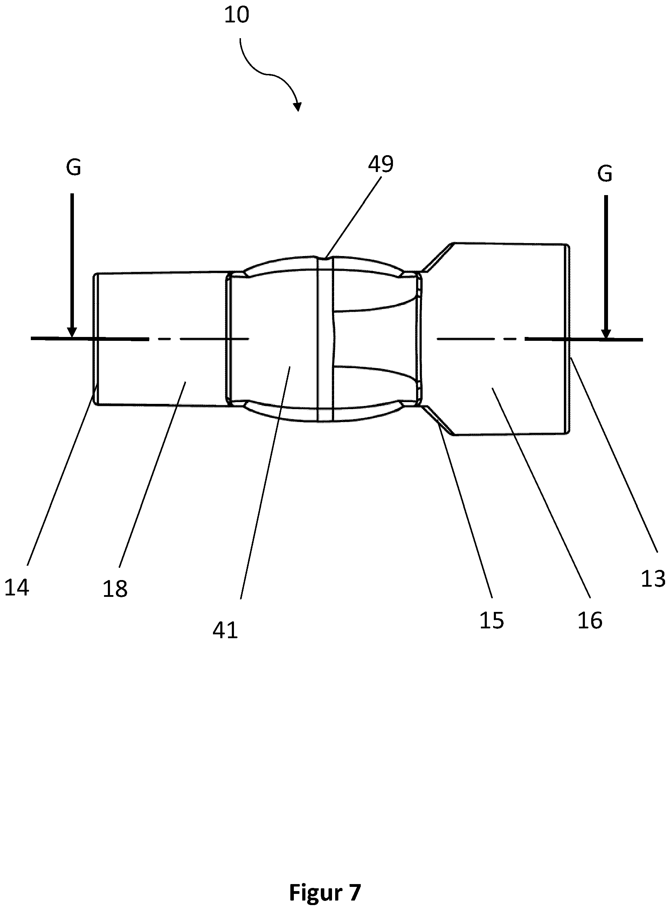

Figur 6 zeigt einen erfindungsgemäßen Adapter 10 in Draufsicht von oben und Figur 7 zeigt einen erfindungsgemäßen Adapter 10 in Draufsicht von unten. Figur 6 verdeutlicht, dass die Tiefe des Grundkörpers T20 wie oben beschrieben schmaler ist als die Aufnahme-Seitenwände 42, 43 und der Aufnahme-Boden 48. Ferner wird deutlich, dass die Aufnahme-Rastungen 44 an den Aufnahme-Seitenwänden 42, 43 in Richtung des Grundkörpers 20 angeformt und in Form eines leichten Vorsprunges ausgebildet sind.

Zudem wird aus Figur 6 ersichtlich, dass der Gehäuseeingang 16 einen größeren Durchmesser hat als der Gehäuseausgang 18. Damit der im Querschnitt größere Gehäuseeingang 16 an den Grundkörper 20 anschließen kann, weist der Gehäuseeingang 16 an der der Eintrittsöffnung 13 abgewandten Seite mindestens eine Abflachung 15 auf.

Ferner ist gezeigt, dass in diesem Ausführungsbeispiel der Griff 41 breiter ausgebildet ist als die Aufnahmevorrichtung 40. Die verbreiterte Ausbildung des Griffes 41 erleichtert das Ergreifen des Adapters 10. Ein Griff 41 in anderer Ausgestaltung ist ebenfalls möglich.

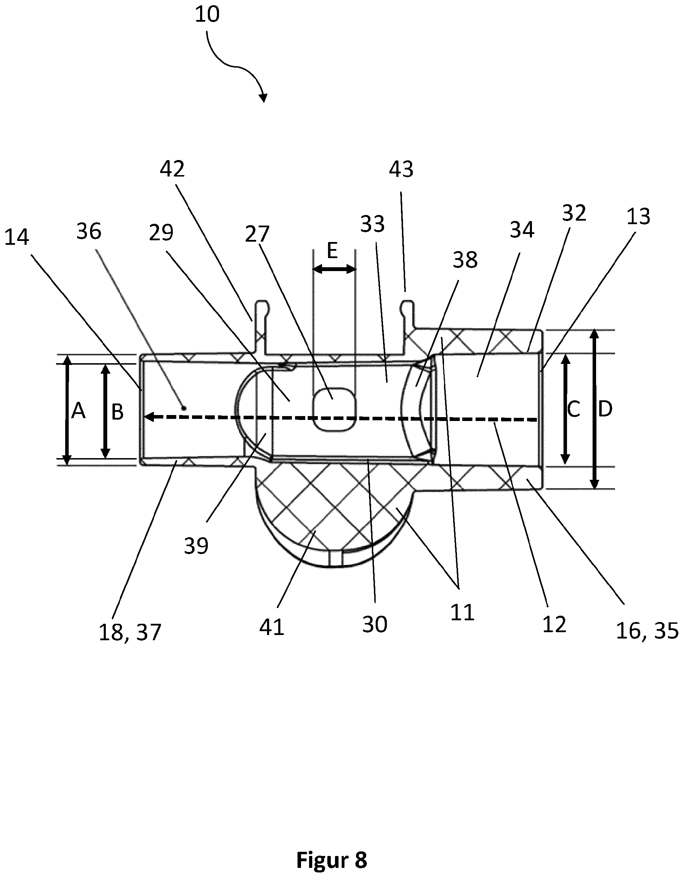

Den Figuren 6 und 7 ist die Schnittebene G für die nachfolgend beschriebene Figur 8 zu entnehmen.

**Figur 8** zeigt einen Längsschnitt nach Linie G-G durch einen erfindungsgemäßen Adapter 10.

Es wird ersichtlich, dass der Durchgangskanal 12 durch das Gehäuse 11 begrenzt wird. Das Gehäuse 11 umschließt den Adapter 10 in seiner gesamten Länge L10 (siehe Fig. 1). Der Durchgangskanal 12 erstreckt sich somit durch die gesamte Länge L10 des Gehäuses 11.

Das Gehäuse 11 umfasst eine Gehäuseeingangs-Wandung 35, eine Grundkörper-Wandung 30 und eine Gehäuseausgangs-Wandung 37, die den Durchgangskanal 12 begrenzen.

Die Gehäuseeingangs-Wandung 35 umschließt den länglichen Hohlkörper des Gehäuseeingangs 16, der beispielsweise kreisrund ausgebildet ist. Die Gehäuseeingangs-Wandung 35 umschließt einen Gehäuseausgangs-Innenraum 36. Der Gehäuseeingang 16 hat beispielsweise einen konstanten Innendurchmesser, der einem Innendurchmesser C der Eintrittsöffnung 13 entspricht. Der Innendurchmesser kann im Verlauf auch größer oder kleiner werden.

Die Gehäuseausgangs-Wandung 37 umschließt den länglichen Hohlkörper des Gehäuseausgangs 18, der beispielsweise kreisrund ausgebildet ist. Die Gehäuseausgangs-Wandung 37 umschließt einen Gehäuseausgangs-Innenraum 36. Der Gehäuseausgang 18 hat beispielsweise einen konstanten Innendurchmesser, der einem Innendurchmesser B der Austrittsöffnung 14 entspricht. Der Innendurchmesser kann im Verlauf auch größer oder kleiner werden.

Die Grundkörper-Wandung 30 umschließt den Hohlkörper des Grundkörpers 20, der beispielsweise als Vierkantrohr mit planparallelen Flächen ausgebildet ist. Die Grundkörper-Wandung 30 umschließt einen Grundkörper-Innenraum 29. Der Grundkörper 20 ist beispielsweise konstant in seiner Höhe H20 und seiner Tiefe T20, so dass der Durchgangskanal 12 im Bereich des Grundkörpers 20 konstant ausgebildet ist.

Der Durchgangskanal 12 erstreckt sich von der Eintritts-Öffnung 13 über den Gehäuseeingangs-Innenraum 34, den Grundkörper-Innenraum 29 und den Gehäuseausgangs-Innenraum 36 zu der Austritts-Öffnung 14. Der Innendurchmesser des Durchgangskanals 12 kann konstant groß ausgebildet sein. In der abgebildeten beispielhaften Ausführungsform ist der Durchmesser des Durchgangskanals 12 beispielsweise nicht konstant.

Beispielsweise ist ein Innendurchmesser C der Eintrittsöffnung 13 größer als ein Innendurchmesser B der Austrittsöffnung 14. Der Innendurchmesser C der Eintrittsöffnung 13 liegt in einem Bereich zwischen 25 und 5 mm. Der Innendurchmesser C der Eintrittsöffnung 13 beträgt beispielsweise 15,56 mm ± 0,05. Der Innendurchmesser B der Austrittsöffnung 14 liegt in einem Bereich zwischen 25 und 5 mm. Der Innendurchmesser B der Austrittsöffnung 14 beträgt beispielsweise 12,4 mm ± 0,05.

Die Wandstärke des Gehäuseeingangs 16 und des Gehäuseausgangs 18 können gleich oder unterschiedlich stark ausgestaltet sein. Im vorliegenden Ausführungsbeispiel ist die Wandstärke der Gehäuseeingangs-Wandung 35 stärker ausgebildet als die Wandstärke der Gehäuseausgangs-Wandung 37. Dadurch ergibt sich ein gegenüber dem Außendurchmesser A der Austrittsöffnung 14 größerer Außendurchmesser D der Eintrittsöffnung 13.

Der Außendurchmesser D der Eintrittsöffnung 13 liegt in einem Bereich zwischen 35 und 5 mm. Der Außendurchmesser D der Eintrittsöffnung 13 beträgt beispielsweise 22,01 mm ± 0,05. Der Außendurchmesser A der Austrittsöffnung 14 liegt in einem Bereich zwischen 35 und 5 mm. Der Außendurchmesser der Austrittsöffnung A beträgt beispielsweise 15,23 mm ± 0,05.

Der Durchgangskanal 12 kann durch die jeweiligen Begebenheiten in den unterschiedlichen Bauteilen unterschiedliche Querschnitte aufweisen. Der Querschnitt des Durchgangskanals 12 kann in manchen Ausführungsformen auch konstant sein.

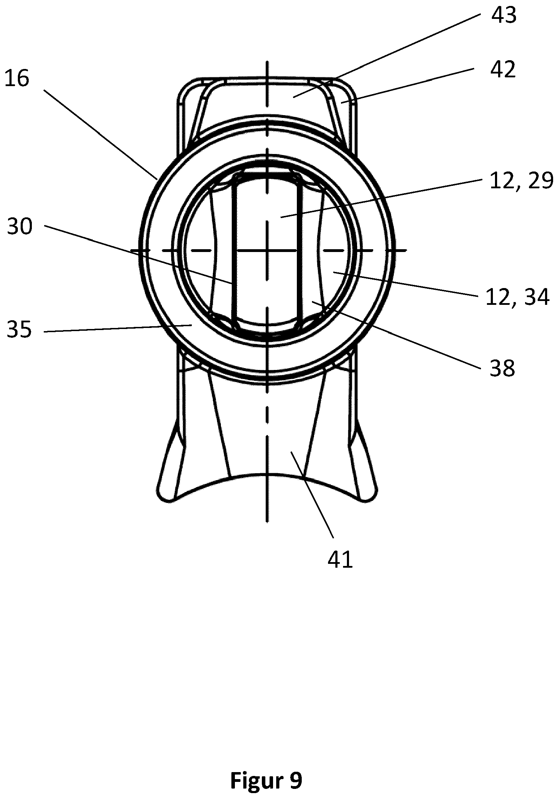

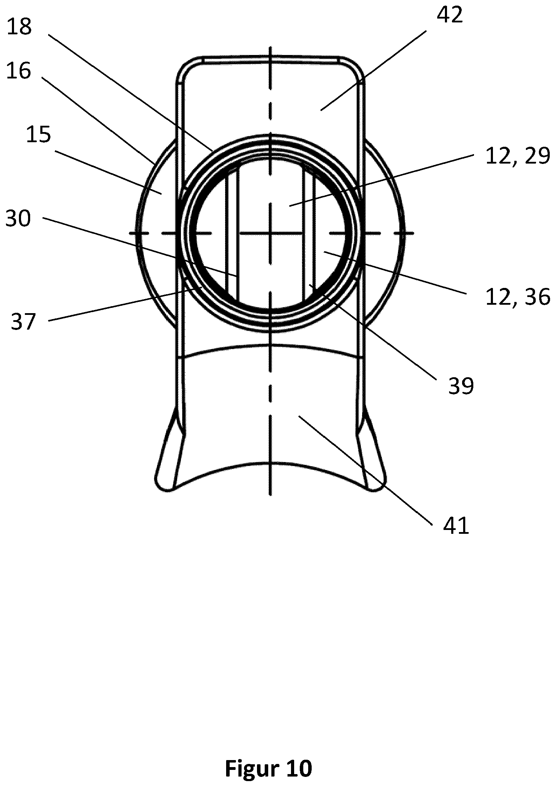

In der vorliegenden beispielhaften Ausführungsform hat der Durchgangskanal 12 im Bereich des Gehäuseeingangs 16 und des Gehäuseausgangs 18 beispielsweise einen runden Querschnitt (siehe unten, Fig. 9) und im Bereich des Grundkörpers 20 beispielsweise einen viereckigen Querschnitt (siehe unten, Fig. 10).

Der Verlauf des Durchgangskanals 12 vom Gehäuseeingangs-Innenraum 34 zum Grundkörper-Innenraum 29 erfolgt sukzessive über einen Eingangs-Übergang 38. Der Eingangs-Übergang 38 ist trichterförmig ausgebildet, so dass sich eine sukzessive Verengung und Änderung des Querschnittes des Durchgangskanals 12 ergibt. Durch den trichterförmigen Verlauf des Eingangs-Übergangs 38 wird die Strömungseigenschaft des Durchgangskanals 12 optimiert und Verwirbelungen und/oder Störungen des Gasflusses vermieden.

Der Verlauf des Durchgangskanals 12 vom Grundkörper-Innenraum 29 zum Gehäuseausgangs-Innenraum 36 erfolgt über einen Ausgangs-Übergang 39. Der Ausgangs-Übergang 39 kann trichterförmig ausgebildet sein. In dem abgebildeten Ausführungsbeispiel ist der Ausgangs-Übergang nicht trichterförmig ausgebildet. Zwischen dem eckigen Querschnitt des Grundkörpers 20 und dem kreisrunden Querschnitt des Gehäuseausganges 18 ist beispielsweise keine trichterförmige Verengung vorgesehen.

Der in Fig. 8 dargestellte Längsschnitt nach Linie G-G zeigt ferner, dass die Gehäuse-Innenwandung 32 glatt ausgebildet ist. Eine glatte Gehäuse-Innenwandung 32 vermindert Verwirbelungen oder Störungen des Gasflusses. Hervorzuheben ist, dass die Gehäuse-Innenwandung 32 auch im Bereich der Seitenwand 33 glatt ausgebildet ist. Es ist im inneren des Grundkörpers 20 keine Profilierung ausgebildet. Die Gehäuse-Innenwand 32 ist durchbrochen von mindestens zwei seitlichen Öffnungen 26, 27, von denen im Längsschnitt nur eine abbildbar ist. Die Öffnung 26, 27 ist, wie weiter oben hierin beschrieben, in Form eines Polygons, beispielsweise als quadratische Öffnung 26, 27mit abgerundeten Ecken ausgebildet.

Die quadratische Öffnung 26, 27 ist in Ihrer Höhe und Breite E maximal 15 mm groß, bevorzugt weniger als 10 mm. In einer beispielhaften Ausführungsform liegt die Höhe und Breite E in einem Bereich von 8 bis 1 mm. Das Öffnungsmaß E beträgt beispielsweise 5,88 mm ± 0,20.

**Figur 9** zeigt einen erfindungsgemäßen Adapter 10 in Draufsicht von der Gehäuseeingangsseite 16 aus. Aus Fig. 9 ist ersichtlich, dass der Gehäuseeingang 16 im dargestellten Ausführungsbeispiel einen kreisrunden Querschnitt hat. Der Durchgangskanal 12 erstreckt sich durch den kreisrunden Hohlraum des Gehäuseeingangs-Innenraums 34.

In diesem Ausführungsbeispiel ist der Eingangs-Übergang 38 abgerundet ausgebildet. Durch den Eingangs-Übergang 38 ist ein trichterförmiger Verlauf des Durchgangskanals 12 vom Gehäuseeingangs-Innenraum 34 zum Grundkörper-Innenraum 29 ausgebildet. So ist der Querschnitt des Durchgangskanals 12 im Gehäuseeingangs-Innenraum 34 kreisrund und im Grundkörper-Innenraum 29 viereckig ausgebildet.

Figur 9 verdeutlicht ferner, dass die Aufnahme-Seitenwand 43 sich nach oben hin verjüngt, während die Aufnahme-Seitenwand 42 in der Breite gleichbleibt. Diese Lösung ermöglicht die Kompatibilität des Adapters 10 mit CO₂ -Sensoren verschiedener Hersteller.

**Figur 10** zeigt einen erfindungsgemäßen Adapter 10 in Draufsicht von der Gehäuseausgangsseite 18 aus. Aus Fig. 10 ist ersichtlich, dass der Gehäuseausgang 18 im dargestellten Ausführungsbeispiel einen kreisrunden Querschnitt hat. Weiterhin wird ersichtlich, dass der Gehäuseausgang 18 in diesem Ausführungsbeispiel einen kleineren Durchmesser als der Gehäuseeingang 16 aufweist. Der Durchgangskanal 12 erstreckt sich durch den kreisrunden Hohlraum des Gehäuseausgangs-Innenraums 36. Der Durchgangskanal 12 erstreckt sich ferner durch den viereckigen Hohlraum des Grundkörper-Innenraums 29. Im dargestellten Ausführungsbeispiel ist der Ausgangs-Übergang 39 nicht abgerundet oder trichterförmig ausgebildet.

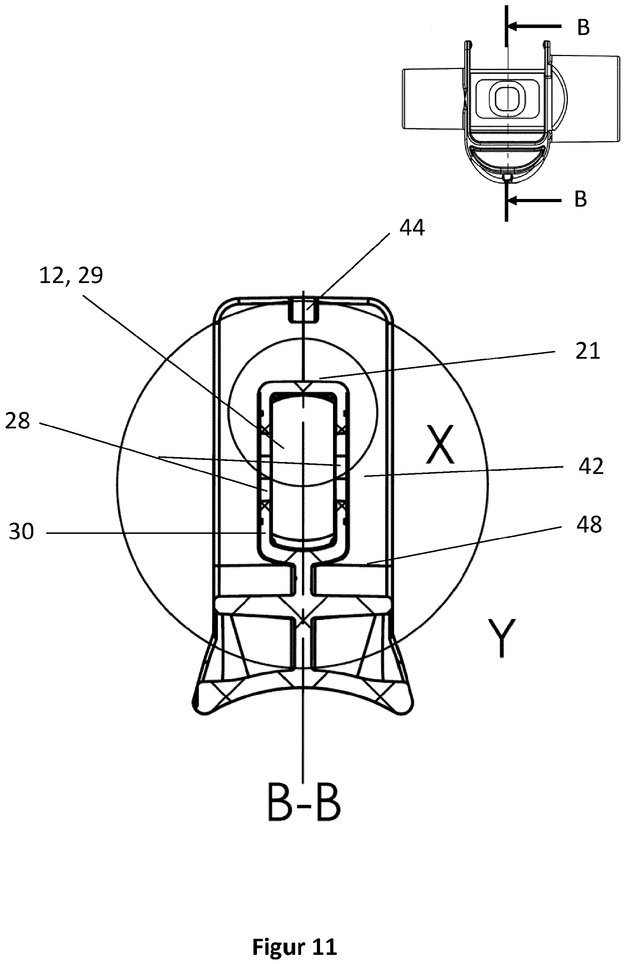

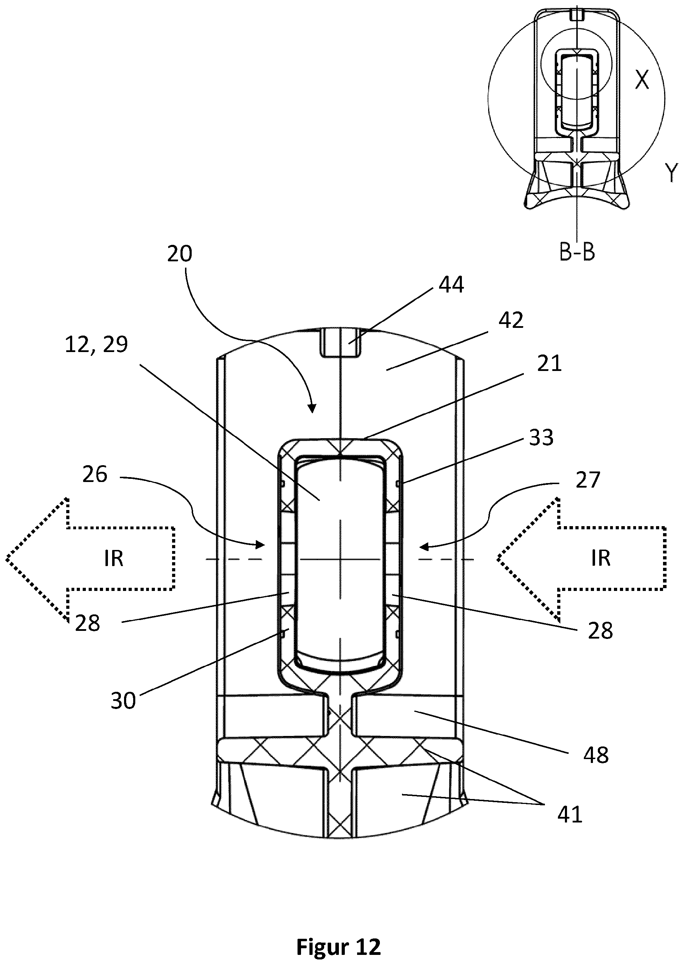

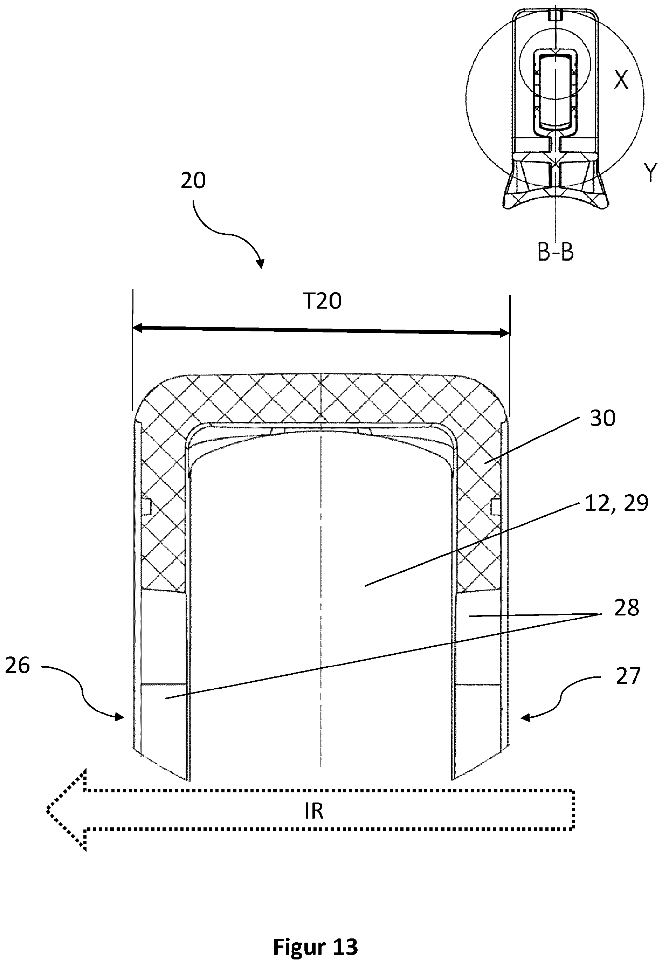

**Figur 11** zeigt einen Querschnitt nach Linie B-B, der im Wesentlichen durch den Grundkörper 20 eines erfindungsgemäßen Adapters 10 führt. **Figur 12** zeigt eine Detailansicht des Schnitts nach Linie B-B im Bereich Y. **Figur 13** zeigt eine Detailansicht des Schnitts nach Linie B-B im Bereichs X.

Die Figuren 11 bis 13 zeigen beispielhaft im Detail, wie der Bereich, in dem die Messung mit dem nicht gezeigten IR-Spektrometer 80 ausgebildet sein kann.

Fig. 12 zeigt, dass der Grundkörper 20 im Wesentlichen planparallele Seitenwände 33 hat. Die Grundkörper- Wandung 30 ist unterbrochen von mindestens zwei Öffnungen 26, 27. Diese werden durch die Laibungen 28 eingefasst. Durch die beiden Öffnungen 26, 27 kann Strahlung, z.B. IR-Strahlung derart durch den Grundkörper 20 gerichtet werden, dass die IR-Strahlung den Grundkörper-Innenraum 29 und somit auch den Durchgangskanal 12 schneidet. Die IR-Strahlung kann durch die Öffnung 27 in den Grundkörper-Innenraum 29 hineingeleitet und durch die Öffnung 26 aus dem Grundkörper-Innenraum 29 hinausgeleitet werden. Die IR-Strahlung kann auch umgekehrt verlaufen, also von der Öffnung 26 zu der Öffnung 27. Dadurch, dass die IR-Strahlung den Durchgangskanal 12 schneidet, können Gase, beispielsweise Atemgase, die sich in dem Durchgangskanal 12 befinden und/oder durch diesen hindurchgeführt werden, spektrometrisch analysiert werden.

Fig. 13 zeigt eine Detailansicht des Schnitts nach Linie B-B im Bereichs X. Die Grundkörper- Wandung 30 ist unterbrochen von den zwei Öffnungen 26, 27, die durch die Laibungen 28 eingefasst werden (Teilabbildung). Es ist schematisch angedeutet, wie die IR-Strahlung den Grundkörper 20 in seiner gesamten Tiefe T20 schneidet. Hierbei kreuzt die IR-Strahlung auch das sich im Durchgangskanal 12 befindliche Atemgas.

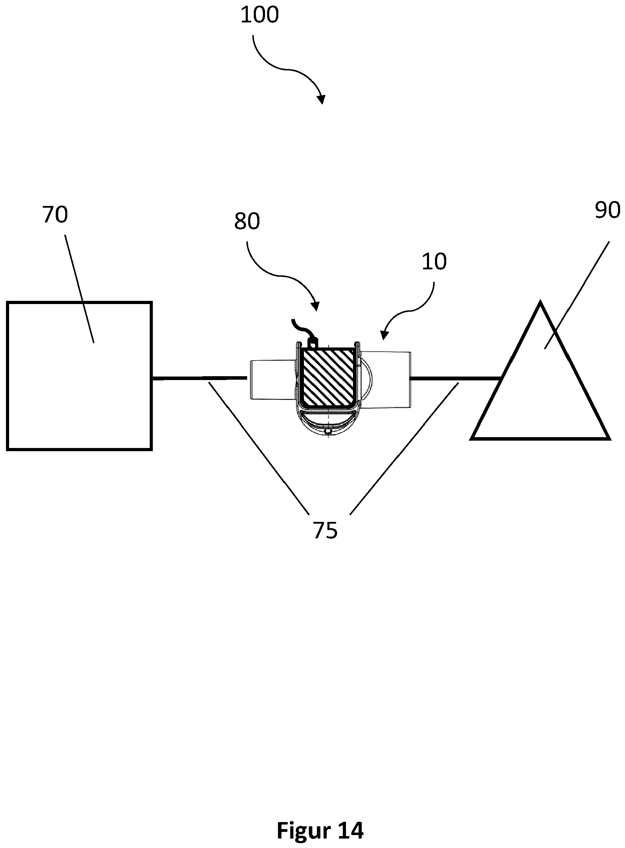

Der erfindungsgemäße Adapter 10 eignet sich beispielsweise für die Verwendung mit einem System 100 zur Analyse eines Atemgasstromes, also für die Verwendung mit einem Beatmungsgerät 70 und/oder einem Patienteninterface 90 und einem IR-Spektrometer. In dem System 100 kann ein oder mehrere Adapter 10 verwendet werden, wobei der mindestens eine Adapter 10 gasleitend mit dem Patienteninterface 90 und/oder dem Beatmungsgerät 70 verbunden ist. Fig. 14 zeigt beispielhaft eine schematische Übersicht über ein System 100 mit einem erfindungsgemäßen Adapter 10 mit aufgesetztem IR-Spektrometer 80, der über eine Leitung 75 mit einem Beatmungsgerät 70 und einem Patienteninterface 90 gasleitend verbunden ist.

Der erfindungsgemäße Adapter 10 ist mit einem Patienteninterface 90 verwendbar. Als Patienteninterface 90 ist jegliches Peripheriegerät zu verstehen, welches zur Interaktion mit einem Lebewesen ausgebildet ist. Insbesondere ist das Patienteninterface 90 zu Therapie- oder Diagnosezwecken in Verbindung mit dem Adapters 10 und/oder mit dem Beatmungsgerät 70 ausgebildet. Das Patienteninterface 90 kann als Maske ausgebildet sein. Diese Maske kann eine Full-Face Maske, also Nase und Mund umschließend sein, oder eine Nasenmaske, also eine nur die Nase umschließende Maske. Auch Trachealtuben bzw. -kanülen und sogenannte Nasenbrillen können als Patienteninterface 90 eingesetzt werden. In manchen Fällen kann das Patienteninterface 90 auch ein einfaches Mundstück, beispielsweise ein Rohr oder Schlauch sein, durch welches das Lebewesen ausatmet und/oder einatmet.

Der Adapter 10 kann im und/oder am Patienteninterface 90 angeordnet sein. Der Adapter 10 kann in manchen Ausführungsformen direkt in das Patienteninterface 90 integriert sein. In anderen Ausführungsformen können Adapter 10 und Patienteninterface 90 direkt miteinander koppelbar sein. Adapter 10 und Patienteninterface 90 können auch über mindestens eine Leitung 75 miteinander verbunden sein.

Der erfindungsgemäße Adapter 10 ist sowohl mit einem Beatmungsgerät 70 als auch ohne ein Beatmungsgerät 70 verwendbar. Unter einem Beatmungsgerät 70 sind Geräte zu verstehen, die einen Anwender oder Patienten bei der natürlichen Atmung unterstützen und/oder die Beatmung eines Anwenders oder Patienten übernehmen und/oder einer Atemtherapie dienen und/oder anderweitig auf die Atmung eines Anwenders oder Patienten einwirken. Darunter fallen zum Beispiel, aber nicht ausschließlich, CPAP- sowie BiPAP-Geräte, Narkose- bzw. Anästhesiegeräte, Atemtherapiegeräte, klinische, außerklinische oder Notfall-Beatmungsgeräte, Highflow-Therapiegeräte und Hustenmaschinen.

Beatmungsgeräte 70 können auch als Diagnosegeräte für die Beatmung verstanden werden. Diagnosegeräte können dabei allgemein zur Erfassung von medizinischen, physiologischen und/oder atmungsbezogenen Parametern eines Lebewesens dienen. Darunter fallen auch Geräte, welche medizinische Parameter von Patienten in Kombination mit der Atmung oder ausschließlich die Atmung betreffend, erfassen und optional verarbeiten können.

Der erfindungsgemäße Adapter 10 kann im und/oder am Beatmungsgerät 70 angeordnet sein. Der Adapter 10 kann in manchen Ausführungsformen direkt in das Beatmungsgerät 70 integriert sein. In anderen Ausführungsformen können Adapter 10 und Beatmungsgerät 70 direkt miteinander koppelbar sein. Adapter 10 und Beatmungsgerät 70 können auch über mindestens eine Leitung 75 miteinander verbunden sein.

Patienteninterface 90 und/oder Beatmungsgerät 70 und/oder Adapter 10 sind bevorzugt über mindestens eine Leitung 75 miteinander verbunden. Die Leitung 75 ist als Gasleitung ausgebildet, die die einzelnen Komponenten des Systems 100 miteinander verbindet. Die Leitung 75 ist dabei bevorzugt so ausgebildet, dass es zu keiner ungewollten Leckage kommt. Ferner ist die Verbindung bevorzugt flexibel und/oder drehbar ausgebildet. Die Leitung 75 kann beispielsweise als elastisches Rohr und/oder Schlauch und/oder Schlauchsystem ausgebildet sein.

Der erfindungsgemäße Adapter 10 kann zu Analysezwecken beispielsweise zwischen einem Patienteninterface 90 und einem Beatmungsgerät 70 angeordnet werden, sodass das Patienteninterface 90 über den Adapter 10 mit dem Beatmungsgerät 70 gasleitend verbunden ist (siehe Fig. 14).

Zur Analyse des Atemgases wird der erfindungsgemäße Adapter 10 beispielsweise in Verbindung mit einem IR-Spektrometer 80 verwendet. Das IR-Spektrometer 80 kann mit einem Sensor IR-Strahlung aussenden. Diese IR-Strahlung wird, wie hierin ausführlich dargestellt, durch den erfindungsgemäßen Adapter 10 geleitet, wobei die IR-Strahlung das Atemgas schneidet. Anschließend wird die IR-Strahlung von einem Empfänger empfangen. Eine Analyseeinrichtung kann die empfangene IR-Strahlung analysieren und optional über ein Kabel zur Datenübertragung auf einen Datenträger übertragen.

Obwohl die vorliegende Erfindung anhand der Ausführungsbeispiele detailliert beschrieben wurde, ist es für den Fachmann selbstverständlich, dass die Erfindung nicht auf diese Ausführungsbeispiele beschränkt ist. Vielmehr sind Abwandlungen in einer Weise möglich, dass einzelne Merkmale weggelassen werden oder andersartige Kombinationen der beschriebenen Einzelmerkmale verwirklicht werden können, sofern der Schutzbereich der beiliegenden Ansprüche nicht verlassen wird. Die vorliegende Offenbarung schließt sämtliche Kombinationen der vorgestellten Einzelmerkmale mit ein.

### Bezugszeichenliste

- 10: Adapter
- H10: Höhe des Adapters
- L10: Länge des Adapters
- 11: Gehäuse
- 12: Durchgangskanal
- 13: Eintrittsöffnung
- 14: Austrittsöffnung
- 15: Abflachung
- 16: Gehäuseeingang
- 18: Gehäuseausgang
- 20: Grundkörper
- H20: Höhe des Grundkörpers
- L20: Länge des Grundkörpers
- T20: Tiefe des Grundkörpers
- 21: Oberseite
- 22: Fase
- 23: Seitenfläche
- 24: Äußerer Rand
- 25: Innerer Rand
- 26: Öffnung
- 27: Öffnung
- 28: Laibung
- 29: Grundkörper-Innenraum
- 30: Grundkörper-Wandung
- 31: Unterseite
- 32: Gehäuse-Innenwandung
- 33: Seitenwand
- 34: Gehäuseeingangs-Innenraum
- 35: Gehäuseeingangs-Wandung
- 36: Gehäuseausgangs-Innenraum
- 37: Gehäuseausgangs-Wandung
- 38: Eingangs-Übergang
- 39: Ausgangs-Übergang
- 40: Aufnahmevorrichtung
- 41: Griff
- 42: Aufnahme-Seitenwand
- 43: Aufnahme-Seitenwand
- 44: Aufnahme-Rastung
- 48: Aufnahme-Boden
- 49: Anspritzpunkt
- 60: Abdeckung / Folie
- 62: Seitenlänge
- 64: Ecke
- 70: Beatmungsgerät
- 75: Leitung
- 80: IR-Spektrometer
- 90: Patienteninterface
- 100: System
- A: Außendurchmesser der Austrittsöffnung
- B: Innendurchmesser der Austrittsöffnung
- C: Innendurchmesser der Eintrittsöffnung
- D: Außendurchmesser der Eintrittsöffnung
- E: Öffnungs-Maß
- O: Oben
- U: Unten

## Patentansprüche

1. Adapter 10 zur Verwendung mit Spektrometern 80 für die Analyse von Gasen mit einem Gehäuse 11, das einen Grundkörper 20, eine Aufnahmeeinrichtung 40 zur Aufnahme eines Spektrometers 80, einen Gehäuseeingang 16 und einen Gehäuseausgang 18 umfasst, wobei der Gehäuseeingang 16 eine Eintrittsöffnung 13 und der Gehäuseausgang 18 eine Austrittsöffnung 14 umfasst, und mit einem Durchgangskanal 12, der zwischen der Eintrittsöffnung 13 und der Austrittsöffnung 14 angeordnet ist und wobei der Grundkörper 20 zwischen dem Gehäuseeingang 16 und dem Gehäuseausgang 18 angeordnet ist **dadurch gekennzeichnet, dass** der Grundkörper 20 mindestens zwei Öffnungen 26, 27 aufweist, die beidseitig des Durchgangskanals 12 an sich gegenüberliegen Seiten angeordnet sind.

2. Adapter 10 nach Anspruch 1, **dadurch gekennzeichnet, dass** der Grundkörper 20 zwei Seitenwände 33 mit jeweils einer Öffnung 26, 27 aufweist, durch die eine Strahlung derart gerichtet werden kann, dass die Strahlung den Durchgangskanal 12 schneidet, wobei die Strahlung eine Infrarot-Strahlung ist.

3. Adapter 10 nach einem der vorherigen Ansprüche, **dadurch gekennzeichnet, dass** der Adapter eine maximale Länge L10 aufweist, wobei sich der Durchgangskanal 12 von der Eintrittsöffnung 13 zu der Austrittsöffnung 14 über die maximale Länge L10 des Adapters 10 erstreckt, wobei der Durchgangskanal 12 ausgebildet und eingerichtet ist, Atemgase zu führen.

4. Adapter 10 nach einem der vorherigen Ansprüche, **dadurch gekennzeichnet, dass** die Öffnungen 26, 27 in ihrer Grundform ein Polygon sind, bevorzugt ein Viereck.

5. Adapter 10 nach einem der vorherigen Ansprüche, **dadurch gekennzeichnet, dass** mindestens eine der mindestens zwei Öffnungen 26, 27 zur Herstellung eines Betriebsmodus mit mindestens einer Abdeckung 60 abgedeckt werden.

6. Adapter 10 nach einem der vorherigen Ansprüche, **dadurch gekennzeichnet, dass** Adapter 10 und Abdeckung 60 einstückig oder zweistückig ausgeführt sind, wobei Adapter 10 und Abdeckung 60 aus demselben Material oder aus unterschiedlichen Materialien bestehen.

7. Adapter 10 nach einem der vorherigen Ansprüche, **dadurch gekennzeichnet, dass** Adapter 10 und Abdeckung 60 zweistückig ausgeführt und aus demselben Materialien gefertigt sind.

8. Adapter 10 nach einem der vorherigen Ansprüche, **dadurch gekennzeichnet, dass** Adapter 10 und Abdeckung 60 aus einem Kunststoff gefertigt sind, bevorzugt aus einem thermoplastischen Kunststoff.

9. Adapter 10 nach einem der vorherigen Ansprüche, **dadurch gekennzeichnet, dass** das Material des Adapters 10 und der Abdeckung 60 ausgewählt ist aus der Gruppe Polycarbonate (PC), Polymethylmethacrylate (PMMA), Polystyrole (PS), Cycloolefin-Copolymere (COC, Topas).

10. Adapter 10 nach einem der vorherigen Ansprüche, **dadurch gekennzeichnet, dass** der Adapter 10 und die Abdeckung 60 aus PMMA Plexiglas 7N, Topas 6017-S04, PC Lexan 121 RM1, PC Makrolon 2400, PS Styrolution 124 L, Topas 8007 oder COC 8007 X-04, bevorzugt aus COC oder PMMA gefertigt sind.

11. Adapter 10 nach einem der vorherigen Ansprüche, **dadurch gekennzeichnet, dass** die Abdeckung 60 für Strahlung, insbesondere IR-Strahlung, durchlässig ist.

12. Adapter 10 nach einem der vorherigen Ansprüche, **dadurch gekennzeichnet, dass** die Abdeckung 60 eine Durchlässigkeit für Infrarotstrahlung von mindestens 0,5 aufweist.

13. Adapter 10 nach einem der vorherigen Ansprüche, **dadurch gekennzeichnet, dass** die Abdeckung 60 eine Folie 60 ist.

14. Adapter 10 nach einem der vorherigen Ansprüche, **dadurch gekennzeichnet, dass** die Folie 60 eine Dicke zwischen 2000 µm und 50 µm, bevorzugt zwischen 1000 µm und 100 µm, besonders bevorzugt zwischen 600 µm und 100 µm, beispielsweise 140 µm aufweist.

15. Adapter 10 nach einem der vorherigen Ansprüche, **dadurch gekennzeichnet, dass** die Folie 60 größer ist als die Öffnung 26, 27.

16. Adapter 10 nach einem der vorherigen Ansprüche, **dadurch gekennzeichnet, dass** der Grundkörper 20 zwei Seitenwände 33 umfasst, die jeweils eine Öffnung 26,27, einen inneren Rand 25, einen äußeren Rand 24 und eine Seitenfläche 23 umfassen, wobei die Folie 60 mindestens bereichsweise auf die Seitenwände 33 aufgebracht wird und mindestens eine der mindestens zwei Öffnungen 26, 27 zur Herstellung eines Betriebsmodus des Adapters 10 vollständig abdeckt.

17. Adapter 10 nach einem der vorherigen Ansprüche, **dadurch gekennzeichnet, dass** die Folie 60 die Öffnungen 26, 27 derart abdeckt, dass der Durchgangskanal 12 luftdicht abgeschlossen ist und ein Durchgang von Gasen ausschließlich von dem Gehäuseeingang 16 zu dem Gehäuseausgang 18 möglich ist und umgekehrt.

18. Adapter 10 nach einem der vorherigen Ansprüche, **dadurch gekennzeichnet, dass** die Folie 60 verklebt, verschweißt und/oder mechanisch verrastet mit der Seitenwand 33 oder Teilen der Seitenwand 33 verbunden wird.

19. Adapter 10 nach einem der vorherigen Ansprüche, **dadurch gekennzeichnet, dass** 20 % bis 80 % der Folie 60 mit der Seitenwand 33 oder mit Teilen der Seitenwand 33 verbunden werden, bevorzugt 30 % bis 60 %, besonders bevorzugt 40 % bis 50 %.

20. Adapter 10 nach einem der vorherigen Ansprüche, **dadurch gekennzeichnet, dass** die Folie 60 zumindest bereichsweise mit dem inneren Rand 25 und/oder dem äußeren Rand 24 und/oder der Seitenfläche 23 verbunden wird.

21. Adapter 10 nach einem der vorherigen Ansprüche, **dadurch gekennzeichnet, dass** die Folie 60 und der innere Rand 25 und/oder der äußeren Rand 24 und/oder die Seitenfläche 23 über Laserschweißen verbunden werden.

22. Adapter 10 nach einem der vorherigen Ansprüche, **dadurch gekennzeichnet, dass** die Folie 60 eine polygone Form aufweist, bevorzugt eine viereckige Form.

23. Adapter 10 nach einem der vorherigen Ansprüche, **dadurch gekennzeichnet, dass** die Folie 60 Seitenlängen 62 umfasst, wobei die Seitenlängen 62 der Folie 60 eine Länge von 5 mm bis 60 mm aufweisen, bevorzugt von 10 mm bis 30 mm, besonders bevorzugt von 15 bis 20 mm.

24. Adapter 10 nach einem der vorherigen Ansprüche, **dadurch gekennzeichnet, dass** die Aufnahmevorrichtung 40 einen Aufnahme-Boden 48 und zwei Aufnahme-Seitenwände 42, 43 umfasst und die Aufnahmevorrichtung 40 eingerichtet und ausgebildet ist, ein formkomplementäres IR-Spektrometer 80 derart zwischen die zwei Aufnahme-Seitenwände 42, 43 und den Aufnahme-Boden 48 aufzunehmen, dass zumindest die mindestens zwei Öffnungen 26, 27 des Grundkörpers 20 vom IR-Spektrometer 80 umschlossen sind.

25. System 100 zur Analyse eines Atemgasstromes mindestens umfassend ein Beatmungsgerät 70 und/oder ein Patienteninterface 90 und ein IR-Spektrometer 80, **dadurch gekennzeichnet, dass** das System 100 mindestens einen Adapter 10 nach einem der vorhergehenden Ansprüche umfasst, wobei der Adapter 10 mit dem Beatmungsgerät 70 und/oder dem Patienteninterface 90 gasleitend verbunden ist und wobei das IR-Spektrometer 80 den Adapter zumindest bereichsweise derart umschließt, dass IR-Strahlung den Atemgasstrom schneidet.
